# EUROPEAN PATENT SPECIFICATION

(11) **EP 3 891 306 B1**
(45) Date of publication and mention of the grant of the patent: **26.02.2025**
(21) Application number: 19821052.8
(22) Date of filing: 06.12.2019
(51) Int. Cl.: C12Q 1/6883

(54) **BIOMARKER FOR DETECTING ZEARALENONE EFFECT**
BIOMARKER ZUM NACHWEISS EINES ZEARALENON EFFEKTS
BIOMARQUEUR POUR DETECTER L'EFFET DU ZEARALENONE

(30) Priority: 06.12.2018 EP 18210684
(43) Date of publication of application: 13.10.2021
(73) Proprietor: DSM Austria GmbH, 3131 Getzersdorf bei Traismauer (AT)
(72) Inventor: GRENIER, Bertrand, 3071 Böheimkirchen (AT); NAGL, Veronika, 3071 Böheimkirchen (AT); SCHATZMAYR, Gerd, 3430 Tulln (AT); BINDER, Eva-Maria, 3430 Tulln (AT)
(74) Representative: Schiweck Weinzierl Koch Patentanwälte Partnerschaft mbB
(86) International application number: PCT/EP2019/083953
(87) International publication number: WO 2020/115270

(56) References cited:
- US-B2- 8 574 838
- ZHU KEWEI ET AL: "Green tea polyphenol EGCG suppresses osteosarcoma cell growth through upregulating miR-1", TUMOR BIOLOGY, KARGER, BASEL, CH, vol. 37, no. 4, 24 October 2015 (2015-10-24), pages 4373 - 4382, XP036220764, ISSN: 1010-4283, [retrieved on 20151024], DOI: 10.1007/S13277-015-4187-3
- BRZUZAN ET AL: "MicroRNA expression profiles in liver and colon of sexually immature gilts after exposure to Fusarium mycotoxins", POL J VET SCI., vol. 18, no. 1, 1 January 2015 (2015-01-01), pages 29 - 38, XP055581606
- THERMOFISHER SCIENTIFIC: "Advanced miRNA Assays TaqMan Array Plates", 26 October 2016 (2016-10-26), XP055581763, Retrieved from the Internet <URL:https://assets.thermofisher.com/TFS-Assets/LSG/manuals/MAN0016121_TaqManAdvmiRNAArrayPlates_QR.pdf> [retrieved on 20190416]

## Description

### FIELD OF THE INVENTION

The present invention relates to a novel method for detecting zearalenone (ZEN) effect comprising determining the expression level of at least one miRNA in a test sample, as well as to a novel use of at least one miRNA for detecting zearalenone (ZEN) effect in a test sample.

### DESCRIPTION

Mycotoxins are secondary metabolites of molds, which lead to concentration-dependent adverse health effects in animals. In Northern and Central Europe, mycotoxins produced by field fungi such as *Fusarium graminearum* or *F. culmorum* are most frequently detected in animal feeds (Streit et al. 2012. Toxins, 4, 788-809). Zearalenone (ZEN) is regarded as one of the most relevant *Fusarium* mycotoxins, particularly in pig production. ZEN is a frequent contaminant of maize but can also occur in various other commodities such as wheat, barley or oats. In a recent study, 88% of tested feed samples tested positive for ZEN with median and maximum concentrations of 20 µg/kg and 11,192 µg/kg, respectively (Kovalsky et al. 2016. Toxins, 8, E363). Pigs are especially susceptible to the effects of ZEN, which is attributed to the species-specific metabolism of this mycotoxin (Fink-Gremmels and Malekinejad. 2007. Animal Feed Science and Technology, 137, 326-341). ZEN exhibits low acute toxicity but acts as full and partial agonist on estrogen receptors α and β, respectively (Zinedine et al. 2007. Food and Chemical Toxicology, 45, 1-18). As a consequence, ZEN exposure of pigs results in reproduction and fertility disorders. For example, mammary gland development is triggered in prepuberal gilts fed diets containing 1-5 ppm ZEN. Also, vulvovaginitis, edema of vulva and rectal prolapses are observed (Osweiler, G. 1999. Mycotoxins. In: STRAW, B., DÁLLAIRE, S., MENGELING, W. & TAYLOR, D. (eds.) 8th Diseases of swine. Ames, Iowa, USA: Iowa State University Press; Osweiler, G. D. 2000. Mycotoxins: Contemporary issues of food animal health and productivity. Veterinary Clinics of North America: Food Animal Practice, 16, 511-530). In mature sows, the effects of ZEN are dependent on the concentration in the feed, duration of exposure and phase of gestation, and range from anestrus to a decrease in live embryos, an increase in dead-born piglets and abortions (Kordić et al. 1992. Journal of environmental pathology, toxicology and oncology: official organ of the International Society for Environmental Toxicology and Cancer, 11, 53-55). In newborn piglets of exposed sows, enlargement of external genitalia and a higher incidence of piglets with splay leg and trembling were recorded (Osweiler, G. 1999. Mycotoxins. In: STRAW, B., DÁLLAIRE, S., MENGELING, W. & TAYLOR, D. (eds.) 8th Diseases of swine. Ames, Iowa, USA: Iowa State University Press). In addition, ZEN activates different receptors, most prominently the pregnane X receptor, involved in the regulation of cytochrome P450 isoforms in vitro, thus potentially affecting the phase I metabolism of various endo- and xenobiotics (Ayed-Boussema et al. 2011. Environmental Toxicology and Pharmacology, 31, 79-87).

So far, detection of ZEN mycotoxicosis is mainly based on the observation of clinical signs and feed analysis. Yet, ZEN, as many other mycotoxins, exhibits adverse health effects in livestock before clinical symptoms become evident. Analysis of feed samples is accompanied by certain challenges: Representative feed samples need to be collected taking into account an inhomogeneous distribution of mycotoxins within a lot as well as time point of sampling as feed lots might have changed between sampling and the initial onset of mycotoxin-induced effects. Also, mycotoxin co-contamination of feed samples needs be considered, which can result in additive, synergistic or antagonistic effects in animals (Grenier and Oswald. 2011. World Mycotoxin Journal, 4, 285-313). Moreover, the presence of modified forms of ZEN are not routinely monitored but add up to the total ZEN burden on an individuum (Binder et al. 2017. Toxins, 9, 56). In contrast to feed analyses, the detection of specific biomarkers in biological matrices of swine, such as urine, feces, bile or tissue would allow detection of mycotoxicosis on an individual level. Two types of biomarkers are used in mycotoxin research: Exposure-based biomarkers and mechanism-based biomarkers (Baldwin et al. 2011. World Mycotoxin Journal, 4, 257-270). While the former relate to the measurement of a mycotoxin itself and/or its metabolites, the latter relate to a specific biological response, e.g. increase/decrease in protein levels or cellular metabolite levels, that can be linked to mycotoxin intake. Concerning ZEN, efforts have been taken during the last years to establish an exposure-based biomarker in pigs. Under controlled conditions, numerous studies have investigated effects of increasing toxin concentrations on levels of ZEN and/or its metabolites in blood, urine and bile, but also in feces, liquor, liver or back fat. Usually, highest concentrations of ZEN and its metabolites were recovered from bile and urine, with better correlation coefficients, i.e. ingested ZEN versus recovered ZEN, for bile and urine as matrices than for other matrices such as blood (Döll et al. 2003. Archives of Animal Nutrition, 57, 311-334; Goyarts et al. 2007. Food Additives and Contaminants, 24, 369-380; Brezina et al. 2014. Archives of animal nutrition, 68, 425-447). In accordance, a recent field study failed to correlate ZEN levels in sow feed to those in plasma, as toxin concentrations in plasma were under the limit of quantification in the majority of samples (Van Limbergen et al. 2017. Veterinary Record, 181, 539). Exposure-based biomarkers can still not be recommended for a prediction of ZEN intake under field conditions, since comparable concentrations of ZEN in bile, blood or urine can derive from a broad range of ZEN exposure levels (Dänicke and Winkler. 2015. Food and Chemical Toxicology, 84, 225-249). Most of all, ZEN residues do not provide indications on the severity of adverse health effects induced by ZEN, as demonstrated e.g. by the absence of a correlation between the total ZEN levels in bile and the uterus weight after ZEN exposure (Dänicke and Winkler. 2015. Food and Chemical Toxicology, 84, 225-249). Regarding mechanism-based ZEN biomarkers, several biological parameters, such as hematological and biochemical parameters (Goyarts et al. 2007. Food Additives and Contaminants, 24, 369-380; Gaj 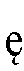 cka et al. 2016. Research in Veterinary Science, 109, 169-180) or mRNA expression (Dai et al. 2016. Animal Reproduction Science, 168, 126-137; Reddy et al. 2017. Asian-Australasian Journal of Animal Sciences, 31, 595-606; Zhou et al. 2018. Asian-Australasian Journal of Animal Sciences, 31, 32), were found altered upon ZEN exposure *in vivo.* However, the reproducibility and specificity of the studied parameters is limited, to the extent that no clear dose-response relationship between the increase/decrease of these biological parameters in blood, urine or bile and ZEN intake could be established so far. He et al. (He et al. 2018. Endocrinology, 159, 2993-3006) describe an increase of the expression of the microRNA (miRNA) miR-7 in pituitary tissue of ovariectomized pigs upon intraperitoneal injection of 112.5 mg ZEN per day. However, He et al. applied an experimental setup that does not reflect natural ZEN exposure due to contaminated feed, since the amount of 112.5 mg ZEN exceeds the realistic exposure level by far. Also, administration by injection does not reflect natural intake with the feed. Further, ovariectomized pigs are not good models for ZEN exposure of healthy, non-ovariectomized pigs, since ZEN exposure commonly correlates with reproduction and fertility disorders. Further, it would be desirable to identify a biomarker that would be useful for detection of ZEN exposure in a more accessible sample than pituitary tissue, such as serum.

Brzuzan et al. (2015-01-01) observed changes in miRNA expression in the liver and the descending colon of patients after exposure to Fusarium mycotoxins.

The present invention was made in view of the prior art outlined above. In general, the inventors of the present application have found that mechanism-based biomarkers would be more appropriate for application in the field for detection of mycotoxicosis than biomarkers of exposure due to simpler detection methods and a direct correlation to mycotoxin effects in an individuum, but so far no suitable biomarker-of-effect or mechanism-based biomarker has been identified.

The present invention provides a method for detecting zearalenone (ZEN) effect, which method comprises determining in a test sample the expression level of at least one miRNA as disclosed in claim 1, and comparing the expression level with the expression level of said at least one miRNA in a control sample. miRNAs (also referred to as microRNAs) are short RNA molecules of a length of commonly from about 19 to about 24 nucleotides. miRNAs are negative regulators of gene expression capable of blocking translation of mRNA into proteins or of degrading mRNA molecules. By providing a method for detecting ZEN effect comprising determining in a test sample the expression level of at least one miRNA described herein; and comparing the expression level with a reference value, the inventors have surprisingly identified a reliable way for detecting ZEN effect via a biomarker-of-effect which is suitable for application in the field.

The term "expression level" of a polynucleotide such as *e.g.* an mRNA molecule or a miRNA, refers to a relative or absolute amount of said polynucleotide. Expression levels of polynucleotides can be determined by appropriate strategies known to a person skilled in the art including but not limited to microarry experiments, quantitative real-time PCR, or so-called next-generation sequencing (NGS) technologies offered *e.g.* by Illumina, Pacific Biosciences, Oxford Nanopore Technologies etc.

Zearalenone (ZEN) is a nonsteroidal macrocyclic lactone with the following structural formula, which can be synthesized by way of the polyketide metabolic pathway: 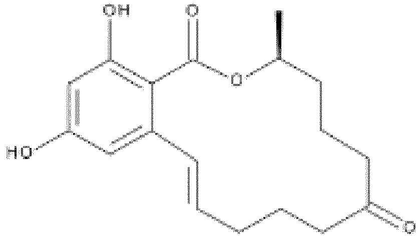 Its name according to the IUPAC nomenclature is (2E,11S)-15,17-dihydroxy-11-methyl-12-oxabicyclo[12.4.0]octadeca-1(18),2,14,16-tetraene-7,13-dione. A variety of ZEN derivatives also occurs in nature and may be formed by enzymatic or chemical modifications of ZEN. Examples include but are not limited to glycosidic ZEN conjugates or those containing sulfate, formed by fungi, plants or a mammalian metabolism as well as ZEN metabolites formed in the human or animal organism, among others. ZEN derivatives are understood below to be ZEN conjugates or ZEN metabolites that occur naturally or are synthesized by chemical or biochemical synthesis but in particular α-zearalenol (α-ZEL; (2E,7R,11S)-7,15,17-trihydroxy-11-methyl-12-oxabicyclo[12.4.0]-octadeca-1(18),2,14,16-tetraen-13-one), β-zearalenol (β-ZEL; (2E,7S,11S)-7,15,17-trihydroxy-11-methyl-12-oxabicyclo[12.4.0]octadeca-1(18),2,14,16-tetraen-13-one), α-zearalanol (α-ZAL; (7R,11S)-7,15,17-trihydroxy-11-methyl-12-oxabicyclo[12.4.0]octadeca-1(18),14, 16-trien-13-one), β-zearalanol (β-ZAL; (7S,115)-7,15,17-trihydroxy-11-methyl-12-oxabicyclo[12.4.0]octadeca-1(14),15,17-trien-13-one), zearalenone 14-sulfate (Z14S; [(2E,11S)-15-hydroxy-11-methyl-7,13-dioxo-12-oxabicyclo[12.4.0]octadeca-1(18),2,14,16-tetraen-17-yl] hydrogen sulfate), zearalenone-14-glycoside (Z14G; (2E,11S)-15-hydroxy-11-methyl-17-[(3R,4S,5S,6R)-3,4,5-trihydroxy-6-(hydroxymethyl)-tetrahydropyran-2-yl]oxy-12-oxabicyclo[12.4.0]octadeca 1(18)2,14,16-tetraene-7,13-dione) as well as zearalanone (ZAN; (11S)-15,17-dihydroxy-11-methyl-12-oxabicyclo-[12.4.0]octadeca-1(18),14,16-triene-7,13-dione). ZEN as well as ZEN derivatives, in particular α-ZEL, β-ZEL, Z14S, α-ZAL, β-ZAL, Z14G and ZAN can also be detected in processed foods and animal feed products, such as bread or beer because of their high chemical and physical stability.

As used herein, the term "zearalenone effect" or "ZEN effect" refers to exposure of a biological system to zearalenone or a (physiological) effect of zearalenone on a biological system. The biological system may be any biological system, such as a cell, a cell culture, a tissue, or an animal. In some embodiments, "zearalenone effect" may further refer to exposure of a biological system to a zearalenone derivative or analog or a (physiological) effect of a zearalenone derivative or analog on a biological system. The zearalenone derivative is preferably one that is disclosed herein above. It is understood that the zearalenone derivative or analog is preferably one that has the same or a similar effect as zearalenone on the biological system. Preferably, "zearalenone effect" relates to zearalenone exposure.

A "test sample" as used herein preferably relates to a sample, for which it should be determined, whether or not it has been contacted to ZEN (or a ZEN analog or derivative) or whether the subject it has been obtained from has been exposed to ZEN (or a ZEN analog or derivative). The methods of the present invention may thus comprise step (b) providing the test sample.

The reference value may correspond to the expression level of said at least one miRNA according to the invention, that has been determined in one or more control samples. Depending on the type of the sample, such a control sample may have been contacted with (preferably a known amount of) ZEN or a ZEN analog or derivative, or a control sample that has not been contacted with ZEN or a ZEN analog or derivative. Such a control sample may have been obtained from a subject that has been exposed to (a preferably known amount of) ZEN or a ZEN analog or derivative, or may have been obtained from a subject that has not been exposed to ZEN or a ZEN analog or derivative. Here, contacting the sample with ZEN or a ZEN analog or derivative that is not bioavailable, e.g. because it is bound to another substance that prevents interaction of ZEN or a ZEN analog or derivative with a biological system, or exposing a subject to ZEN or a ZEN analog or derivative that is not bioavailable, is equivalent to not contacting ZEN or a ZEN analog or derivative with the sample or not exposing the subject with ZEN or a ZEN analog or derivative. The reference value may also be an average or mean value that has been determined in multiple control samples. The at least one miRNA according to the invention, may be selected from the group consisting of sc-miR-1, ssc-miR-181c, ssc-miR-206, ssc-miR- 503, ssc-miR-542-3p, ssc-miR-135, ssc-miR-129a-3p, ssc-miR-142-3p, ssc-miR-432-5p, ssc-miR-455-5p, ssc-miR-182, ssc-miR-493-3p, ssc-miR-455-3p, ssc- miR-183, and ssc-miR-140-3p. The at least one miRNA of the invention may also be a combination of two, three, four, five, six, seven, eight, nine, ten, eleven, twelve, or even more, or all miRNAs selected from aforementioned group.

Throughout the application, unless specified otherwise, miRNAs are denoted by accession numbers of the miRBase sequence database in the version of release 21 of June 2014 (Kozomara A, Griffiths-Jones S. Nucleic Acids Res. 2014 42:D68-D73; Kozomara A, Griffiths-Jones S. Nucleic Acids Res. 2011 39:D152-D157; Griffiths-Jones S, Saini HK, van Dongen S, Enright AJ. Nucleic Acids Res. 2008 36:D154-D158;Griffiths-Jones S, Grocock RJ, van Dongen S, Bateman A, Enright AJ. Nucleic Acids Res. 2006 34:D140-D144; Griffiths-Jones S. Nucleic Acids Res. 2004 32:D109-D111). The following table summarizes some of the miRNAs of the disclosure.

| **Accession No.** | **Sequence** | **SEQ ID NO** |
|---|---|---|
| ssc-miR-1 | uggaauguaaagaaguaugua | 01 |
| ssc-miR-125a | ucccugagacccuuuaaccugug | 02 |
| ssc-miR-125b | ucccugagacccuaacuuguga | 03 |
| ssc-miR-127 | ucggauccgucugagcuuggcu | 04 |
| ssc-miR-129a | aagcccuuaccccaaaaagcau | 05 |
| ssc-miR-133a-5p | agcugguaaaauggaaccaaau | 06 |
| ssc-miR-135 | uauggcuuuuuauuccuauguga | 07 |
| ssc-miR-136 | acuccauuuguuuugaugaugga | 08 |
| ssc-miR-140-3p | uaccacaggguagaaccacggac | 09 |
| ssc-miR-142-3p | uguaguguuuccuacuuuaugg | 10 |
| ssc-miR-143-5p | ggugcagugcugcaucucugg | 11 |
| ssc-miR-146b | ugagaacugaauuccauaggc | 12 |
| ssc-miR-181c | aacauucaaccugucggugagu | 13 |
| ssc-miR-182 | uuuggcaaugguagaacucacacu | 14 |
| ssc-miR-183 | uauggcacugguagaauucacug | 15 |
| ssc-miR-187 | ucgugucuuguguugcagccgg | 16 |
| ssc-miR-195 | uagcagcacagaaauauuggc | 17 |
| ssc-miR-204 | uucccuuugucauccuaugccu | 18 |
| ssc-miR-206 | uggaauguaaggaaguguguga | 19 |
| ssc-miR-22-3p | aagcugccaguugaagaacugu | 20 |
| ssc-miR-22-5p | aguucuucaguggcaagcuuua | 21 |
| ssc-miR-335 | ucaagagcaauaacgaaaaaug | 22 |
| ssc-miR-34a | uggcagugucuuagcugguugu | 23 |
| ssc-miR-369 | aauaauacaugguugaucuuu | 24 |
| ssc-miR-378 | acuggacuuggagucagaaggc | 25 |
| ssc-miR-424-5p | cagcagcaauucauguuuugaa | 26 |
| ssc-miR-432-5p | ucuuggaguaggucauugggu | 27 |
| ssc-miR-450a | uuuugcgauguguuccuaauau | 28 |
| ssc-miR-450b-5p | uuuugcaauauguuccugaaua | 29 |
| ssc-miR-450c-5p | uuuugcgauguguuccuaauac | 30 |
| ssc-miR-455-3p | gcaguccaugggcauauacac | 31 |
| ssc-miR-455-5p | uaugugccuuuggacuacaucg | 32 |
| ssc-miR-486 | uccuguacugagcugccccgag | 33 |
| ssc-miR-493-3p | ugaaggucuacugugugccagg | 34 |
| ssc-miR-493-5p | uuguacaugguaggcuuucauu | 35 |
| ssc-miR-497 | cagcagcacacugugguuugu | 36 |
| ssc-miR-503 | uagcagcgggaacaguacugcag | 37 |
| ssc-miR-542-3p | ugugacagauugauaacugaaa | 38 |
| ssc-miR-708-3p | caacuagacugugagcuucuaga | 39 |
| ssc-miR-758 | uuugugaccugguccacuaac | 40 |
| ssc-miR-7135-3p | aucugucugugucucugagcag | 41 |
| hsa-miR-135b-5p | uauggcuuuucauuccuauguga | 42 |
| hsa-miR-301a-5p | gcucugacuuuauugcacuacu | 43 |
| put-miR-300 | uugcaggaacuugugagucuccua | 44 |

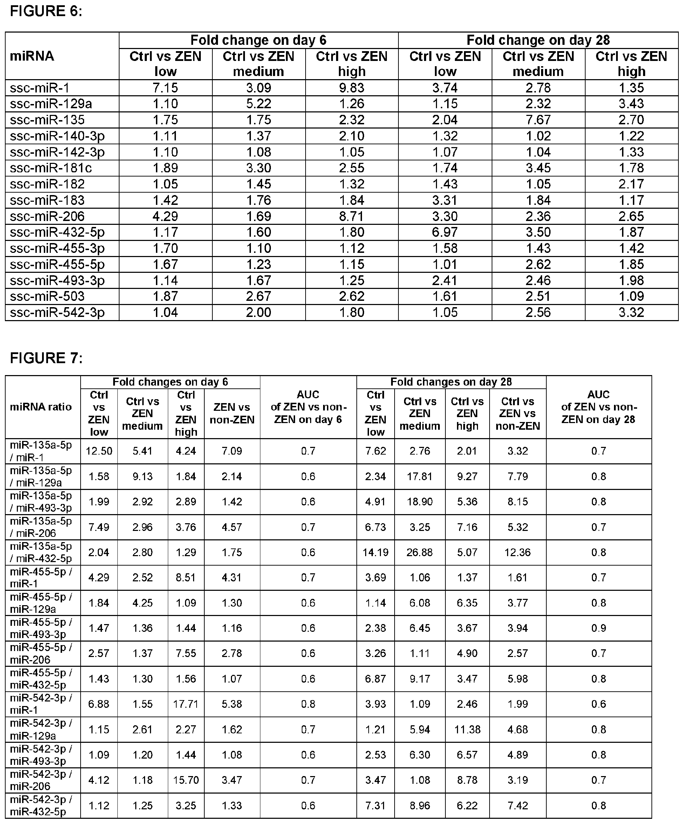

The methods of the present invention may comprise comparing the level of the at least one miRNA determined in a test sample with a reference value. If the at least one miRNA comprises more than one miRNA, comparing the level between these miRNAs may include comparing the ratio between two of the miRNAs. For example, where the at least one miRNA comprise two, three, four, or five miRNAs selected from the group consisting of or comprising at least some, preferably all of ssc-miR-542-3p, ssc-miR-1, ssc-miR-493-3p, ssc-miR-135, ssc-miR-432-5p, and ssc-miR-455-5p, comparing the level may refer to comparing the ratio between ssc-miR-542-3p and ssc-miR-1, ssc-miR-542-3p and ssc-miR-493-3p, ssc-miR-135 and ssc-miR-432-5p, and/or ssc-miR-455-5p and ssc-miR-493-3p. Comparing the level may also refer to comparing the ratio between any of the miRNA pairs shown in Figure 7.

Comparing the level of said at least one miRNA can be based on comparing the individual values of one or more miRNA. For example, a deviation in the expression levels of a miRNA between the test sample and reference value may be indicative for zearalenone effect (such as zearalenone exposure), in particular if the reference value corresponds to the expression level of said miRNA in a control sample that has not been contacted with zearalenone or that has been obtained from a subject that has not been exposed to zearalenone. A deviation in the expression levels may be indicative for zearalenone exposure. The deviation may be expressed in a normal scale, or preferably in a log-fold scale, such as log2-fold scale. The deviation is preferably statistically significant. Several techniques for testing the statistical significance of results are known to the skilled person and it is well within the skill of the person skilled in the art to choose and apply the most appropriate method for analyzing statistical significance. Statistical significance may e.g. be expressed by a p-value or a FDR p adjusted value and the significance level may be ≤0.07, preferably ≤0.05, or more preferably ≤0.001. The deviation may be an increase or decrease. A deviation indicating ZEN exposure may e.g. be at least about 1.3-fold, more preferably at least about 1.5-fold and most preferably at least about 2-fold compared to a reference value corresponding to a sample not having been contacted with zearalenone or obtained from a subject not having been exposed to zearalenone.

Alternatively, where the reference value corresponds to the expression level that has been determined in a control sample that has been contacted with ZEN or a ZEN analog or a ZEN derivative, or has been obtained from a subject that has been exposed to ZEN or a ZEN analog or a ZEN derivative, a non-deviation, a non-significant deviation, or an (untypically) low deviation between the values of test sample and the reference value may be indicative for zearalenone effect, such as zearalenone exposure.

The term "detect" or "detecting" when used herein in the context of detecting zearalenone effect may generally refer to quantitative or qualitative detection. For example, "detect" may refer to qualitative detection, i.e. whether or not a zearalenone effect is present based on the comparison step. "Detect" may also refer to a quantitative detection, i.e. to the extent of the zearalenone effect based on the comparison step.

A "sample" as used herein refers to a biological sample. The sample may comprise a cell. The sample may have been obtained from a subject. Biological samples include, but are not limited to, blood, serum, peripheral blood mononuclear cell (PBMC), urine, feces, semen, or tissue, such as uterine tissue. To allow a rapid detection of ZEN exposure in a subject and thus achieve maximum applicability in the field, a method for detection is preferably applicable to a sample that can be obtained with as little effort as possible. Therefore, the sample is preferably a blood sample, in particular a blood serum sample. By providing a method wherein the sample is a blood serum sample, potential ZEN exposure of a subject can be assessed most conveniently. A blood serum sample can be obtained *e.g.* by punctuation of the *Vena cava cranialis* or the *Vena jugalaris.* Serum samples can be stored at -80 °C for later analysis. A further preferred sample is a uterine sample. A sample may also be a cell culture sample. A cell culture sample may comprise intact cells, but may also comprise or consists of disrupted cells and/or supernatant of intact or disrupted cells. Cells comprised in the cell culture may express estrogen receptors α and/or β, preferably mammalian, such as human or porcine, estrogen receptors α and/or β. Preferred cell lines for a cell culture sample can be MCF-7, T47D, SUM159, HeLa or Ishikawa. Also, primary cells such as e.g. pig granulosa cells can be preferred cells for a cell culture sample.

Where the sample is a uterine tissue sample, the at least one miRNA is preferably selected from the group consisting of ssc-miR-1, ssc-miR-181c, ssc-miR-206, ssc-miR-503, ssc-miR-542-3p, ssc-miR-135, ssc-miR-7135-3p, ssc-miR-204, ssc-miR-143-5p, ssc-miR-187, ssc-miR-335, ssc-miR-424-5p, ssc-miR-450a, ssc-miR-450b-5p, ssc-miR-450c-5p, and ssc-miR-497, wherein the at least one miRNA is preferably ssc-miR-542-3p and/or ssc-miR-1.

Where the sample is a blood sample, such as a serum sample, the at least one miRNA may be selected from the group consisting of ssc-miR-1, ssc-miR-181c, ssc-miR-206, ssc-miR-503, ssc-miR-542-3p, ssc-miR-135, ssc-miR-129a-3p, ssc-miR-142-3p, ssc-miR-432-5p, ssc-miR-455-5p, ssc-miR-182, ssc-miR-493-3p, ssc-miR-455-3p, ssc-miR-183, and ssc-miR-140-3p, preferably selected from the group consisting of ssc-miR-542-3p, ssc-miR-1, ssc-miR-493-3p, ssc-miR-135, ssc-miR-432-5p, and ssc-miR-455-5p.

Comparing the level of said at least one miRNA can also be based on comparing the individual values of multiple miRNAs. Here the relative (or less preferred the absolute) deviations in the expression level in the test sample and the reference value may be combined to yield an even more significant result than comparing the level of only one miRNA. Alternatively, deviation values may be multiplied with or divided by each other, depending on the expected direction of deviation in case of a zearalenone effect.

Comparing the level of said at least one miRNA can also relate to comparison of the ratio of two miRNAs. The ratio may be determined by dividing the level of the one miRNA by the level of the second miRNA as known by those having skill in the art. The inventors found that the determination of the deviation of miRNA ratios as described before allowed surprisingly good discrimination between samples that were exposed to zearalenone (ZEN) and samples that were not exposed to zearalenone (ZEN). If individual measured miRNA are analyzed by qPCR, a possible way to calculate the ratio is by determining ΔCt (e.g. Ct miRNA - Ct UniSp4) for each miRNA to calculate the ratio. As an illustrative example, the ratio between miR-135a and miR-1a may be calculated by ΔCt miRNA 135a - ΔCt miRNA 1a.

Exceptionally good detection of ZEN effect can be achieved when the at least one ratio of expression levels between two miRNAs is selected from the group consisting of the ratios between miR-542-3p and miR-1, miR542-3p and miR493-3p, miR-135a-5p and miR-432-5p, and miR-455-5p and miR-493-3p. A ratio may be considered as being preferred for a method as described herein, if a receiver operating characteristics (ROC) analysis (Grund and Sabin. 2010. Current Opinion in HIV and AIDS, 5, 473-479) to discriminate ZEN exposure vs. non-ZEN exposure results in an area-under-the-curve (AUC)-value that is larger than or equal to 0.7. Alternatively or additionally, the fold change of a miRNA ratio in a ZEN-containing sample is preferably at least about 1.3-fold, more preferably at least about 1.5-fold, most preferably at least about 2-fold compared to a sample not contacted with ZEN or obtained from a subject not having been exposed to ZEN. The fold change can be either positive or negative, i.e. up- or down-regulated compared to a control sample. The level of at least one miRNA even more preferably comprises at least one ratio of expression levels between two miRNAs selected from the group consisting of miR-542-3p and miR-1, miR-542-3p and miR-493-3p, miR-135a-5p and miR-432-5p, and miR-455-5p and miR-493-3p. Hereby it is possible to detect ZEN exposure with especially good reliability and reproducibility.

Comparing the level of said at least one miRNA to a reference value, in particular if multiple miRNA are involved, can also be carried out using complex methods, such as computer-assisted methods, in particular methods involving machine learning. As an illustrative example, an artificial neural network system can be used for comparing the expression level(s) with a reference value. The neural network may be trained by being provided with the value of one or more reference values that may correspond to miRNA levels from one or more samples. Preferably, both, reference values for ZEN exposure and reference values for non-exposure to ZEN may be used for training the neural network. The trained neural network may then be used to detect zearalenone effect. For this purpose, the values of miRNA level(s) of respective miRNA(s) of a test sample may be used as input for the neural network, and the neural network may provide an indication of zearalenone effect, such as a qualitative information, a quantitative information, a score, a probability or any other type of information useful in the detection or characterization of a zearalenone effect. Methods of constructing, training, and applying a neural network are well known to the skilled person. Other (computer-assisted) approaches may also be suitable for being applied in the present invention and are also known the skilled person, such as e.g. Bayesian networks or decision trees.

Generally, the outcome of the comparing step (d) may be any type of information indicating whether or not the sample has been contacted with ZEN (or a ZEN analog or derivative) or whether or not the subject the sample has been obtained from has been exposed to ZEN (or a ZEN analog or derivative). The information may be a qualitative information, a quantitative information, a score, a probability or any other type of useful information. The method of the present invention may thus comprise the further step of (e) providing information regarding zearalenone effect. The information may be provided by any suitable mean known to the skilled person.

A "subject" as used herein refers to a vertebrate, preferably a mammal. The term "mammal" as used herein refers to any animal classified as a mammal, including, without limitation, humans, domestic and farm animals, and zoo, sports, or pet animals, such as sheep, dogs, horses, cats, cows, rats, pigs, primates such as cynomolgus monkeys, to name only a few illustrative examples. A preferred subject is of the genus *Sus,* preferably of the species *Sus scrofa.* Another preferred subject is a human.

ZEN effect, in particular exposure to ZEN, manifests itself as adverse health effects, such as reproduction and fertility disorders, especially in mammalian subjects and in a particularly dramatical manner in subjects of the genus *Sus.* To allow a most rapid detection of ZEN effect in said subjects, the present invention also relates to a method as described above, wherein the subject is preferably a mammal, more preferably of the genus *Sus.*

Methods of the present invention may be for diagnosis, in particular diagnosis of zearalenone exposure of the subject. Such methods may have the purpose of determining a medical condition of a subject in order to decide whether or not or what type of measure should be taken in order to maintain or improve the health of the subject.

Methods of the present invention may also be non-diagnostic. As an illustrative example, a non-diagnostic method can be a feed sampling method. Feed sampling is usually not very accurate due to the non-homogenous distribution of mycotoxins in the feed and typical sample sizes used for this analysis. Due to the comparably large amount of feed uptake by a subject, such as a pig, determining ZEN exposure of the subject may give a more reliable test result of whether or not the feed is contaminated with zearalenone than analyzing the feed directly. Methods of the present invention may therefore be methods for detection of zearalenone in feed or food. Methods of the present invention may comprise prior to obtaining the test sample from the subject step (a) feeding the subject with feed or food suspected to contain zearalenone.

To avoid the undesirable consequences of prolonged exposure to zearalenone, it may become necessary to treat ZEN contaminated feed or food with a ZEN neutralizing agent or to apply a ZEN neutralizing method, in case zearalenone effect is detected in a subject. Therefore, the present invention further relates to a method as described above, wherein the method is for selecting food or feed for contacting with a zearalenone neutralizing agent or for applying a zearalenone neutralizing method. By applying the method described for selecting food or feed for contacting with a ZEN neutralizing agent or for applying a ZEN neutralizing method, adverse health effects can be at least mitigated or completely avoided. Methods of the present invention may thus comprise step (a) feeding the subject with feed suspected to contain zearalenone prior to obtaining the test sample from the subject. Methods of the present invention may also comprise step (f) selecting the feed for contacting with a zearalenone neutralizing agent or applying a zearalenone neutralizing method if a deviation in the expression levels of the at least one miRNA between the test sample and the control sample indicates zearalenone exposure. Methods of the present invention may thus comprise providing a zearalenone neutralizing agent or applying a zearalenone neutralizing method. Methods of the present invention may also comprise contacting the feed or food selected by the method with the zearalenone neutralizing agent or applying the zearalenone neutralizing method to the feed.

A zearalenone neutralizing agent or method may refer to an agent or method that degrades zeralenone, e.g. by altering its molecular structure, which can be the effected by chemical, enzymatic, or physical interaction with the zearalenone neutralizing agent. A less toxic molecule may be the result of such a treatment. A zearalenone neutralizing agent or method may also reduce bioavailability of zearalenone, e.g. making zearalenone unavailable to the subject, preferably to the subject's digestion system. This may be achieved, for example by an agent that binds or adsorbs zearalenone. In general, a zearalenone neutralizing agent may be any suitable type of agent, which may be selected from the group consisting of one or more polypeptide(s), one or more microorganism(s), and one or more zearalenone-binding agent(s).

A zearalenone neutralizing agent may also be an α/β-hydrolase capable of transforming zearalenone or its derivatives or analogs. The person skilled in the art knows various α/β-hydrolases, which are *inter alia* described in Lenfant et al. (2013) 'ESTHER, the database of the α/β-hydrolase fold superfamily of proteins: tools to explore diversity of functions' Nucleic Acids Research, Volume 41, Issue D1, D423-D429 and Mindrebo et al. (2016) 'Unveiling the functional diversity of the Alpha-Beta hydrolase fold in plants' Curr Opin Struct Biol. 233-246. In short, all α/β-hydrolases share the feature of a specific fold called α/β-fold (alpha/beta-fold). The α/β- fold is common to a number of hydrolytic enzymes of widely differing phylogenetic origin and catalytic function. The core of each enzyme is an a/β-structure (rather than a barrel), containing 8 β-strands (b1-b8) connected by α-helices (aA-aF) (Ollis et al.

(1992) 'The alpha/beta hydrolase fold' Protein Eng. 5(3):197-211). Therefore, an α/β-hydrolase as described herein can comprise an α/β-fold. An α/β-hydrolase as described herein preferably comprises the α/β-hydrolase core domain consisting of 8 β-strands (b1 - b8) arranged to a central β-sheet and additionally comprises 6 crossover α-helices (aA - aF).

Members of different classes of α/β-hydrolases as well as their structural characteristics are inter alia described in Kourist et al. (2010) 'The alpha/beta-hydrolase fold 3DM database (ABHDB) as a tool for protein engineering.' Chembiochem. 11(12):1635-43).

As enzymes, α/β-hydrolases are often described to be responsible for the hydrolysis of ester and peptide bonds. However, α/β-hydrolases also participate in the breaking of carbon-carbon bonds, decarboxylation reactions and cofactor-independent dioxygenation of heteroaromatic rings. Thus, α/β-hydrolases can include catalytic members (enzymes) in this superfamily. Non-limiting examples are hydrolases (acetylcholinesterase, carboxylesterase, dienelactone hydrolase, lipase, cutinase, thioesterase, serine carboxypeptidase, proline iminopeptidase, proline oligopeptidase, epoxide hydrolase) along with enzymes that require activation of HCN, H₂O₂ or O₂ instead of H₂O for the reaction mechanism (haloalkane dehalogenase, haloperoxidase, hydroxynitrile lyase). Non-catalytic members can include the neuroligins, glutactin, neurotactin, the C-terminal domain of thyroglobulin, yolk proteins, the CCG1-interacting-factor-B and dipeptidylaminopeptidase VI.

The ESTHER database gathers and annotates published information related to gene and protein sequences of this superfamily. Thus, the person skilled in the art can also obtain α/β-hydrolases from ESTHER (http://bioweb.supagro.inra.fr/ESTHER/general?what=index), a database of the α/β-hydrolase-fold superfamily of proteins.

An α/β-hydrolase as used herein may comprise the sequenceof SEQ ID NO: 45 or a sequence having 60% or more sequence identity to the sequence of SEQ ID NO: 45. Thus, any α/β-hydrolase comprising this sequence is embraced by the term α/β-hydrolase.

Additionally or alternatively, an α/β-hydrolase as used herein can also comprise the sequence of SEQ ID NO: 46 or a sequence having 60% or more sequence identity to the sequence of SEQ ID NO: 46. Thus, any α/β-hydrolase comprising this sequence is embraced by the term α/β-hydrolase.

Additionally or alternatively, an α/β-hydrolase as used herein can also comprise the sequence of SEQ ID NO: 47, 48 or 49 or a sequence having 60% or more sequence identity to the sequence of SEQ ID NO: 47, 48, or 49. Thus, any α/β-hydrolase comprising this sequence is embraced by the term α/β-hydrolase.

Additionally or alternatively, an α/β-hydrolase as used herein can also comprise the sequence of SEQ ID NO: 50 or a sequence having 60% or more sequence identity to the sequence of SEQ ID NO: 50. Thus, any α/β-hydrolase comprising this sequence is embraced by the term α/β-hydrolase.

For example, the α/β-hydrolase can comprise a sequence having at least 60 %, 65 %, 70 %, 75 %, 80 %, 85 %, 90 %, 95 %, 97 %, 98 %, 99 % identity to a sequence of SEQ ID NO: 45. Additionally or alternatively, the α/β-hydrolase can comprise a sequence having at least 60 %, 65 %, 70 %, 75 %, 80 %, 85 %, 90 %, 95 %, 97 %, 98 %, 99 % identity to a sequence of SEQ ID NO: 46. Additionally or alternatively, the α/β-hydrolase can comprise a sequence having at least 60 %, 65 %, 70 %, 75 %, 80 %, 85 %, 90 %, 95 %, 97 %, 98 %, 99 % identity to a sequence of SEQ ID NO: 47, 48, and/or 49. Additionally or alternatively, the α/β-hydrolase can comprise a sequence having at least 60 %, 65 %, 70 %, 75 %, 80 %, 85 %, 90 %, 95 %, 97 %, 98 %, 99 % identity to a sequence of SEQ ID NO: 50.

The sequences of SEQ ID NOs: 45-50 are shown in the Table below.

| SEQ ID NO | Sequence |
|---|---|
| 45 | MVTSPALRDVHVPHAYPEQQVDLGEITMNYAEAGDPDRPAVLLIPE QTGSWWSYEEAMGLLSEHFHVYAVDLRGQGRSSWTPKRYSLDNFG NDLVRFIALVVKRPVVVAGNSSGGVLAAWLSAYSMPGQLRGVLCE DPPFFASELVPAHGHSVRQGAGPVFELFRTYLGDQWSVGDWEGFCR AAGASASPMARSFVADGIPQHLQEYDPEWARVFYEGTVGLSCPHER MLGQVKTPVLLTHHMRGIDPETGNLLGALSDEQALRARRLMDSAG VTVDYESVPDASHMMHQSAPARYVEIFTRWAAALAP |
| 46 | MADPAQRDVYVPHAYPEKQADLGEITMNYAEAGEPDMPAVLLIPEQ TGSWWGYEEAMGLLAENFHVYAVDLRGQGRSSWAPKRYSLDNFG NDLVRFIALVVKRPVIVAGNSSGGVLAAWLSAYSMPGQVRGALCED |
| | APFFASELVTTCGHSIRQAAGPMFELFRTYLGDQWSVGDWTGYCRA ADASSSPMARYFVADEIPQHMREYDPEWARAFWEGTVALHCPHEQ LLTQVKTPVLLTHHMRDIDPDTGHLVGALSDEQAARARLLMESAGV KVDYASVPDALHMMHQFDPPRYVEIFTQWAATLAA |
| 47 | MVTSPALRDVHVPHAYPEQQVDLGEITMNYAEAGDPGRPAVLLIPE QTGSWWSYEEAMGLLAEHFHVYAVDLRGQGRSSWTPKRYSLDNFG NDLVRFIALVVRRPVVVAGNSSGGVLAAWLSAYSMPGQIRGVLCED PPFFASELVPAHGHSVRQGAGPVFELFRTYLGDQWSVGDWEGFRSA ADASASPMARSFVADTIPQHLKEYDPEWARAFYEGTVGLNCPHERM LNRVNTPVLLTHHMRGTDPETGNLLGALSDEQAAQVRRLMESAGV KVDYESVPDASHMMHQSDPARYAEILTPWTAALAP |
| 48 | MVTSPALRDVHVPHAYPEQQVDLGEITMNYAEAGDPDRPAVLLIPE QTGSWWSYEEAMGLLAEHFHVYAVDLRGQGRSSWTPKRYSLDNFG NDLVRFIALVVKRPVVVAGNSSGGVLAAWLSAYSMPGQLRGVLCE DPPFFASELVPAHGHSVRQGAGPVFELFRTYLGDQWSVSDWEGFCR AAGASASPMARSFVADGIPQHLKEYDPEWARAFHEGTVGLNCPHER MLGRVNTPVLLTHHMRGTDPETGNLLGALSDEQAAQARLLMESAG VRVDYESVPDASHMMHQSDPARYAEIFTRWAAALAP |
| 49 | MVTSPALRDVHVPHAYPEQQVDLGEITMNYAEAGDPGRPAVLLIPE QTGSWWSYEEAMGLLAEHFHVYAVDLRGQGRSSWTPKRYSLDNFG NDLVRFMALVVRRPVVVAGNSSGGVLAAWLSAYSMPGQIRGVLCE DPPFFASELVPAHGHSVRQGAGPVFELFRTYLGDQWSVGDWEGFRS AAGASASPMARSFVADTIPQHLKEYDPEWARAFYEGTVGLNCPHER MLNRVNTPVLLTHHMRGTDPETGNLLGALSDEQAAQARRLMESAG VKVDYESVPDASHMMHQSDPARYAEILTPWAAALAP |
| 50 | MAEEGTRSEAADAATQARQLPDSRNIFVSHRFPERQVDLGEVVMNF AEAGSPDNPALLLLPEQTGSWWSYEPVMGLLAENFHVFAVDIRGQG RSTWTPRRYSLDNFGNDLVRFIALVIKRPVVVAGNSSGGLLAAWLSA YAMPGQIRAALCEDAPFFASELVPAYGHSVLQAAGPAFELYRDFLG DQWSIGDWKGFVEAAKASPAKAMQLFPTPDEAPQNLKEYDPEWGR AFFEGTVALHCPHDRMLSQVKTPILITHHARTIDPETGELLGALSDLQ AEHAQDIIRSAGVRVDYQSHPDALHMMHLFDPARYAEILTSWSATLP |
| | AND |

The term "identical" or "percent identity" in the context of two or more polypeptide sequences such as SEQ ID NO: 45-50 refers to two or more sequences or subsequences that are the same, or that have a specified percentage of amino acids that are the same (e.g., at least 85 %, 90 %, 95 %, 96 %, 97 %, 98 % or 99 % identity), when compared and aligned for maximum correspondence over a window of comparison, or over a designated region as measured using a sequence comparison algorithm as known in the art, or by manual alignment and visual inspection. Sequences having, for example, 80 % to 95 % or greater sequence identity are considered to be substantially identical. Those having skill in the art will know how to determine percent identity between/among sequences using, for example, algorithms such as those based on CLUSTALW computer program (Thompson Nucl. Acids Res. 2 (1994), 4673-4680) or FASTDB (Brutlag Comp. App. Biosci. 6 (1990), 237-245), as known in the art.

Also available to those having skills in this art are the BLAST and BLAST 2.6 algorithms (Altschul Nucl. Acids Res. 25 (1977), 3389-3402). The BLASTP program for amino acid sequences uses as defaults a word size (W) of 6, an expect threshold of 10, and a comparison of both strands. Furthermore, the BLOSUM62 scoring matrix (Henikoff Proc. Natl. Acad. Sci., USA, 89, (1989), 10915; Henikoff and Henikoff (1992) 'Amino acid substitution matrices from protein blocks.' Proc Natl Acad Sci U S A. 1992 Nov 15;89(22):10915-9) can be used.

For example, BLAST2.6, which stands for Basic Local Alignment Search Tool (Altschul, Nucl. Acids Res. 25 (1997), 3389-3402; Altschul, J. Mol. Evol. 36 (1993), 290-300; Altschul, J. Mol. Biol. 215 (1990), 403-410), can be used to search for local sequence alignments.

A zearalenone neutralizing agent may also be ozone as e.g. described in Qi L et al. Food Addit Contam Part A Chem Anal Control Expo Risk Assess. 2016 Nov;33(11):1700-1710. A zearalenone neutralizing agent may also be a microorganism, such as e.g. a *Clonostachys rosea* as described by Kosawang C et al., Fungal Biol. 2014 Apr;118(4):364-73, or a *Lactobacillus pentosus* as described by Arunrussamee Sangsila et al., Food Control, Volume 62, April 2016, Pages 187-192 or a yeast strain such as Trichosporon mycotoxinivorans MTV as described by Schatzmayr et al. Mycot. Res. 2003 19: 124-128. Further zearalenone neutralizing agents are e.g. described by Zinedine A et al., Food Chem Toxicol. 2007 Jan;45(1):1-18 or Ji C et al., Animal Nutrition Volume 2, Issue 3, 2016, Pages 127-133.

As described above, a zearalenone neutralizing agent may also reduce bioavailability of zearalenone e.g. by binding and/or adsorption. Examples for such agents can include cholestyramine, divinylbenzene-styrene, bentonites, montmorillonite-rich clays, zeolites, organoclays, activated charcoal, yeast or yeast cell wall-derived products.

Zearalenone neutralizing methods may comprise but are not limited to methods of contacting a zearalenone neutralizing agent with the feed. Methods may also comprise heat treatment or ultrasonic treatment.

The assessment of the efficacy and/or efficiency of mycotoxin neutralizing agents or mycotoxin neutralizing methods poses a considerable challenge due to the lack of suitable methods to allow a determination of whether or not the applied mycotoxin neutralizing agent or method achieved the desired the effect. Methods of the present invention may thus also serve the purpose of assessing the capacity of a method or (test) compound to neutralize ZEN. With other words a method of the invention may be for assessment of the capacity of a method or compound to neutralize ZEN. Such a compound may be selected from the group consisting of one or more polypeptide(s), one or more microorganism(s), including live, dead, lyophilized, autolyzed and/or dormant microorganism(s), and one or more zearalenone-binding agent(s), including organic and/or anorganic zearalenone-binding agent(s). By using the methods of the present invention, the assessment of the efficacy and/or efficiency of a ZEN neutralizing agent or method can be achieved in a particularly fast and quick manner.

In cases where a sample has been obtained from a subject, the method of the invention may thus comprise prior to obtaining the test sample from the subject step (a3) feeding the feed contacted with zearalenone and the test compound to the subject. In such a case, the method may further comprise steps of contacting the feed with zearalenone, contacting the feed with the test compound, and/or contacting zearalenone with the test compound. In general, such steps can be performed in any order. Accordingly the method may comprise prior to step (a3) steps of: (a1) contacting the feed with zearalenone, and (a2) contacting the feed contacted with zearalenone with the test compound; or (a1') contacting the feed with the test compound, and (a2') contacting the feed contacted with the test compound with zearalenone; or (a1") contacting the zearalenone with the test compound, and (a2") contacting the zearalenone contacted with the test compound with the feed.

In some cases, which may be the case if the test sample is a cell culture, the sample may have been directly contacted with zearalenone and the test compound. The control sample used in such a method may be a negative control sample or positive control sample. In negative control sample used in this context refers to a sample that has been contacted with zearalenone and optionally a compound that does not neutralize zearalenone. A positive control sample used in this context refers to a sample that has been contacted with zearalenone and a compound that neutralizes zearalenone, or a sample that has not been contacted with zearalenone. In such a method, the absence of a deviation or a non-significant deviation in the expression levels between the test sample and the positive control sample may be indicative for the capacity of the test compound to neutralize zearalenone. Alternatively or additionally, the presence of a (preferably significant) deviation in the expression levels between the test sample and the negative control sample may be indicative for the capacity of the test compound to neutralize zearalenone. In such methods, the sample may be a sample that has not been obtained from a subject.

In order to avoid time-, labor- and cost-intensive animal trials, research experiments are preferrably performed in cultured cells. The test sample, positive control sample, and/or negative control sample may therefore be a cell culture. As an illustrative example, the cell culture may comprise MCF-7, T47D, SUM159, HeLa or Ishikawa. Also, primary cells such as e.g. pig granulosa cells may be comprised in the cell culture. By providing a method as described herein, wherein the test sample, the control sample, such as the positive control sample or the negative control sample is a cell culture, it is possible to obtain valuable data, *e.g.* on the capacity of a method or compound to neutralize ZEN, in a particularly efficient manner.

The present invention also relates to a use of at least one miRNA according to the inventon for detecting zearalenone effect in a test sample. The at least one miRNA may be selected from the group consisting of sc-miR-1, ssc-miR-181c, ssc-miR-206, ssc-miR- 503, ssc-miR-542-3p, ssc-miR-135, ssc-miR-129a-3p, ssc-miR-142-3p, ssc-miR- 432-5p, ssc-miR-455-5p, ssc-miR-182, ssc-miR-493-3p, ssc-miR-455-3p, ssc- miR-183, and ssc-miR-140-3p. The use may be in any method of the invention. Such a use may be for the purpose of detecting zearalenone exposure of a subject, assessing the capacity of a test compound or a method to neutralize zearalenone, or selecting a food for treatment with a zearalenone neutralizing agent or method. By using at least one miRNA as described herein for detecting zearalenone effect in a test sample, ZEN effect can be detected with especially high reliability and specificity.

To allow a rapid reaction such as *e.g.* the supply with a counteracting agent in case of ZEN exposure, fast availability of test results is desired. This can for example be achieved if determination of the expression level of at least one miRNA in a test sample and comparing the expression level with a reference value is performed with an integrated system. The methods of the invention may thus be computer-implemented methods, which may be carried out by an apparatus for carrying out such a method.

Thus, the present invention also relates to a data processing system comprising a processor configured to perform a method comprising the steps of comparing the expression level of at least one miRNA according to the invention determined in a test sample obtained from a subject with a reference value. Input regarding the expression level of the at least one miRNA is required. The information may be transmitted to the data processing system via a device that is capable of determining such information, e.g. over some transmission medium, such as over electrical wiring or cabling, through fiber optics, or via electromagnetic radiation. Such a device may be integrated in the data processing system or connected to it. Information regarding the expression level of the at least one miRNA may also be transmitted to the data processing system via input through a user interface. If the reference value corresponds to the expression level of said miRNA in a control sample that has been obtained from a subject that has not been exposed to zearalenone, a (significant) deviation in the expression levels of a miRNA between the test sample and reference value may be indicative for zearalenone effect (such as zearalenone exposure). In such a case the method may comprise indicating zearalenone exposure of the subject if a deviation is determined. Alternatively, if the reference value corresponds to the expression level that has been determined is a control sample that has been obtained from a subject that has been exposed to zearalenone, no deviation between the values of test sample and the reference value may be indicative for zearalenone effect, such as zearalenone exposure. In such a case the method may comprise indicating zearalenone exposure of the subject if no deviation or no significant deviation is determined. "No deviation" in this context may also encompass a non-significant deviation, or an (untypically) low deviation. The at least one miRNA may be selected from the group consisting of sc-miR-1, ssc-miR-181c, ssc-miR-206, ssc-miR- 503, ssc-miR-542-3p, ssc-miR-135, ssc-miR-129a-3p, ssc-miR-142-3p, ssc-miR- 432-5p, ssc-miR-455-5p, ssc-miR-182, ssc-miR-493-3p, ssc-miR-455-3p, ssc- miR-183, and ssc-miR-140-3p.

Ideally, the data processing system is connected to or integrated in a device that is capable of determining the expression level of at least one miRNA according to the invention in a test sample. The device may provide the data processing system with information regarding the expression level of said at least one miRNA. Such a device may for example be a sequencing device, such as a quantitative real-time sequencing device. Sequencing devices are known to the skilled person and are e.g. commercially available by Illumina, Pacific Biosciences, Oxford Nanopore Technologies etc. Particularly useful are portable sequencing devices such as a MinION sequencing (Oxford Nanopore Technologies). A device that is capable of determining the expression level of at least one miRNA according to the invention may also be a microarray reader.

Accordingly, the present invention also relates to a sequencing device capable of determining the level of at least one miRNA according to the present invetion, comprising the data processing system of the invention. The at least one miRNA may be selected from the group consisting of sc-miR-1, ssc-miR-181c, ssc-miR-206, ssc-miR- 503, ssc-miR-542-3p, ssc-miR-135, ssc-miR-129a-3p, ssc-miR-142-3p, ssc-miR- 432-5p, ssc-miR-455-5p, ssc-miR-182, ssc-miR-493-3p, ssc-miR-455-3p, ssc- miR-183, and ssc-miR-140-3p.

The present invention also relates to a computer program containing instructions to cause the data processing system disclosed herein or the sequencing device disclosed herein to execute the steps of (a) comparing the expression level of at least one miRNA according to the invention determined in a test sample obtained from a subject with a reference value and (b) indicating zearalenone exposure of the subject if a deviation is determined, wherein reference value corresponds to the expression level that has been determined in a control sample that has been obtained from a subject that has not been exposed to zearalenone; or indicating zearalenone exposure of the subject if no deviation is determined, wherein the reference value corresponds to the expression level that has been determined in a control sample that has been obtained from a subject that has been exposed to zearalenone. When the computer program is loaded into and executed by a data processing system, the data processing system becomes an apparatus for carrying out the methods described herein.

The computer program can be a computer program product accessible from a computer-usable or computer-readable medium providing program code for use by or in connection with a computer or any instruction execution system. The present invention thus also relates to a computer-readable medium having stored thereon the computer program disclosed herein. A computer-usable or computer readable medium can be any apparatus that may include, store, communicate, propagate, or transport the program for use by or in connection with the instruction execution system, apparatus, or device. The medium can be an electronic, magnetic, optical, electromagnetic, infrared, semiconductor, or paper system (or apparatus or device) or a propagation medium. The medium may be any suitable tangible medium, such as a diskette, a CD, a DVD, a BD, a hard drive, a memory stick, a memory card or any other computer readable storage medium.

In order to achieve particularly cost-effective and fast detection of ZEN exposure, a kit is provided for performing the method of detecting ZEN effect in the sense of the present invention, comprising a specific binding agent for at least one miRNA of the invention comprised on or conjugated to a solid support. The at least one miRNA may be selected from the group consisting of sc-miR-1, ssc-miR-181c, ssc-miR-206, ssc-miR- 503, ssc-miR-542-3p, ssc-miR-135, ssc-miR-129a-3p, ssc-miR-142-3p, ssc-miR- 432-5p, ssc-miR-455-5p, ssc-miR-182, ssc-miR-493-3p, ssc-miR-455-3p, ssc- miR-183, and ssc-miR-140-3p. The kit may comprise an unlabeled specific binding partner and a labeled specific binding partner for the at least one miRNA. Preferably, the unlabeled specific binding partner is conjugated to the solid support. The kit may comprise specific binding partners for no more than 1, 2, 3, 4, 5, 6, 7, 8, 9, 10, 11, 12, 13, 14, 15, 16, 17, 18, 19, 20, 21, 22, 23, 24, 25, or 26 miRNAs. In some applications, the kit may comprise specific binding partners for no more than 24, 48, 96, or 384 miRNAs. The kit may comprise specific binding partners for no more than 1, 2, 3, 4, 5, 6, 7, 8, 9, 10, 11, 12, 13, 14, 15, 16, 17, 18, 19, 20, 21, 22, 23, 24, 25, or 26 miRNAs according to methods of the invention. The kit may only comprise specific binding partners for miRNAs according to the methods of the invention.

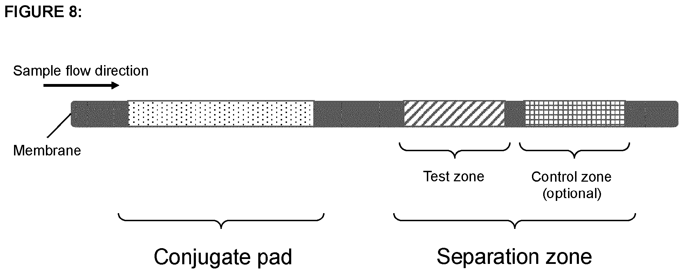

Such a kit may be for a lateral flow test. A schematic representation of a lateral flow device is shown in Fig. 8. The kit may comprise a lateral flow device (LFD), which comprises a carrier (solid support), which has at least two zones, a conjugate pad and a test zone. The conjugate pad comprises a labeled binding agent for the at least one miRNA according to the invention, that can be mobilized in a solvent (e.g. a solvent that comprises target molecule(s)) and be transported therein towards the test zone. The test zone comprises an un-labelled binding agent that is also capable of binding a target molecule. Hereby, targets (the at least one miRNA) that are also bound by the labelled agent from the conjugate pad are immobilized in the test zone. An optional additional control zone may comprise another immobilized binding agent, capable of binding the labelled binding agent from the conjugate pad which was not retained in the test zone. Examples of lateral flow devices are disclosed in Koczula and Gallotta. Essays Biochem. 2016 Jun 30;60(1):111-20 or Krska and Molinelli. Anal Bioanal Chem. 2009 Jan;393(1):67-71.

Also, immunochemical detection could be done by ELISA, e.g. Sandwich-ELISA. The kit may thus comprise a (preferably unlabeled) specific binding agent for the target miRNA that can be immobilized on a solid support or is immobilized on a solid support. The test sample may be added on to the support. Further, a labelled binding agent for the target miRNAs can be added. The miRNA-bound label may then be used for quantification of the miRNA.

The specific binding agent may for example be a nucleic acid, an antibody, an antigen binding fragment of an antibody, or a proteinaceous molecule having antibody-like properties. Where both, a labeled and an unlabeled specific binding partner, are used in the kit, it is preferred that both binding partners can simultaneously bind to the target miRNA. This may for example be achieved if one specific binding partner can bind (e.g. hybridize) to one part of the target miRNA, while the other specific binding partner can bind (e.g. hybridize) to another part of the target miRNA.

The kit may further comprise means for detecting a miRNA that is bound to a specific binding agent, such as an unlabeled specific binding agent described herein, which is preferably conjugated to a solid support. Such means may comprise a probe conjugated to a detectable label. For example, such means may be a labeled specific binding agent as described herein. In general, such a "detectable label" may be any appropriate chemical substance or enzyme, which directly or indirectly generates a detectable compound or signal in a chemical, physical, optical, or enzymatic reaction. For example, a fluorescent or radioactive label can be used to generate fluorescence or x-rays as detectable signal. A dye can be used to generate an optical signal. Alkaline phosphatase, horseradish peroxidase and β-galactosidase are examples of enzyme labels (and at the same time optical labels) which catalyze the formation of chromogenic reaction products. Preferably, the label does not negatively affect the characteristics of the binding partner to which the label is conjugated. A preferred detectable label is an optically detectable label, such as a chromophore or a fluorescent label.

It must be noted that as used herein, the singular forms "a", "an" and "the" include plural references and vice versa unless the context clearly indicates otherwise. Thus, for example, a reference to "a miRNA" or "a process" includes one or more of such miRNAs or processes, respectively, and a reference to "the process" includes equivalent steps and processes that could be modified or substituted known to those of ordinary skill in the art. Similarly, for example, a reference to "miRNAs" includes "a miRNA".

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

The term "more than" includes the concrete number. For example, "more than 20" means ≥20.

Throughout this specification and the claims or items, unless the context requires otherwise, the word "comprise" and variations such as "comprises" and "comprising" will be understood to imply the inclusion of a stated integer (or step) or group of integers (or steps). It does not exclude any other integer (or step) or group of integers (or steps). When used herein, the term "comprising" can be substituted with "containing", "composed of", "including", "having" or "carrying."

The term "about" or "approximately" as used herein means within 20%, preferably within 10%, and more preferably within 5% of a given value or range. It includes, however, also the concrete number, e.g., about 20 includes 20.

When used herein "consisting of" excludes any element, step, or ingredient not specified in the claim element. When used herein, "consisting essentially of" does not exclude materials or steps that do not materially affect the basic and novel characteristics of the claim.

In each instance herein any of the terms "comprising", "consisting essentially of" and "consisting of" may be replaced with either of the other two terms. The possibility to replace terms with each other is not to be understood that these terms are necessarily synonymous.

It should be understood that this invention is not limited to the particular methodology, protocols, material, reagents, and substances, etc., described herein and as such can vary. The terminology used herein is for the purpose of describing particular embodiments only, and is not intended to limit the scope of the present invention, which is defined solely by the claims.

For further clarification of all aspects of the invention, the invention is described in the following by means of figures and examples. Therein:
**FIG. 1** shows the average vulva surface areas of the animals (n=6) of the four treatment groups over the time course of the experiment.
**FIG. 2** shows the number of differentially expressed miRNAs in the uterus samples of ZEN-exposed piglets (with adjusted p-values ≤0.07).
**FIG. 3A** **and** **B** show fold change values of *S. scrofa*-specific miRNAs in uterus tissue significantly affected *(i.e.* with FDR p adjusted value ≤0.07) in at least one ZEN group upon ZEN exposure. The fold change values can be either positive or negative, i.e. up- or down-regulated compared to a control sample.
**FIG. 3C-3E** show fold change values of S. *scrofa*-specific miRNAs in uterus tissue that were not significantly affected *(i.e.* with FDR p adjusted value >0.07). The fold change values can be either positive or negative, i.e. up- or down-regulated compared to a control sample.
**FIG. 4** shows log2-transformed fold changes and expression levels (measured as Tags Per Million, TPM) of putative and predicted miRNAs significantly increased or decreased (FDR p adjusted value ≤0.07) upon ZEN exposure in uterus tissue.
**FIG. 5** shows the results of targeted qPCR-based serum analysis on day 6 and day 28, comparing the Ctrl and ZEN high group. Only miRNAs with quantifiable levels in at least four individuals in each treatment group were considered.
**FIG. 6** shows fold change values of miRNAs from the targeted qPCR-based serum analysis. The fold change values can be either positive or negative, i.e. up- or down-regulated compared to a control sample.
**FIG. 7** shows fold change values of miRNA ratios from the targeted qPCR-based serum analyis of all three ZEN groups as well as a combined value of the three ZEN groups vs control *(i.e.* ZEN vs non-ZEN). Ratios were considered suitable when the area under the curve (AUC) value was ≥0.7 for at least one sample day. The fold change values can be either positive or negative, i.e. up- or down-regulated compared to a control sample.
**FIG. 8** shows a schematic representation of a lateral flow device.

### EXAMPLES

### Example 1: Animals and study design

Weaned female piglets (sow: Landrace x Large White, boar: Pietrain; 30 +/- 2 days old) were obtained from a local producer and individually tagged. Piglets were housed in slatted floor pens under controlled environmental conditions. After an acclimatization period of seven days, twenty-four female piglets were selected based on both body weight and vulva size, and allocated to one of four groups (6 animals/group). Piglets received either blank feed (negative control; Ctrl) or feed contaminated with ZEN in three increasing concentrations (ZEN low: 0.17 mg/kg diet; ZEN medium: 1.5 mg/kg diet; ZEN high: 4.6 mg/kg diet). During the 28-day experimental period, each treatment group was given free access to water and the assigned diet. The pigs were observed daily and weighed weekly.

Diets were manufactured at BIOMIN AN (Herzogenburg, Austria), and formulated according to the energy and amino acid requirements for piglets. Four different batches were prepared, one control batch and three batches artificially contaminated with ZEN (Fermentek LTD, Israel; purity > 99.2%). Homogenous distribution of ZEN within the diets was achieved by preparation of maltodextrin premixes, which contained appropriate amounts of ZEN and were added to the basal feed at an inclusion rate of 0.9% (w/w). Final ZEN concentrations were verified by HPLC-MS (Romer Labs GmbH) and confirmed.

Blood was collected weekly (d 0, 6, 14, 21 and 28) from individual animals by punctuation of the *vena cava cranialis* (Primavette^{®} EDTA, Kabe Labortechnik GmbH, Nümbrecht-Elsenroth, Deutschland). After centrifugation (1500 x g, 20 min), serum samples were stored at -80 °C for later analysis. Vulva length and width were measured at regular intervals with a caliper to determine the dietary ZEN effects. The vulva area was calculated as vulva length × vulva width, as previously reported (Jiang et al. 2011. Journal of animal science, 89, 3008-3015). Eight measurements were done per piglet over the experimental period.

After 28 days of dietary exposure to the experimental diets, pigs were anesthetized by intramuscular injection of ketamine (0.1 mL/kg body weight (b.w.) Narketan^{®}, Vétoquinol GmbH, Ravensburg, Germany) and azaperone (0.03 mL/kg b.w.; Stresnil^{®}, Janssen-Cilag, Neuss, Germany). Subsequently, animals were euthanized by intracardial injection of T61^{®} (Intervet, Unterschleißheim, Germany; 0.1 ml/kg b.w.). The whole reproductive tract was collected, weighed and examined for any clinical signs. Small pieces of the corpus uteri and the mid-jejunum were dissected, placed into 1 mL of RNAlater, and stored overnight at 4 °C. The next day, all the tissue samples were transferred to -80 °C for long-term storage, and until processed for transcriptomics analysis.

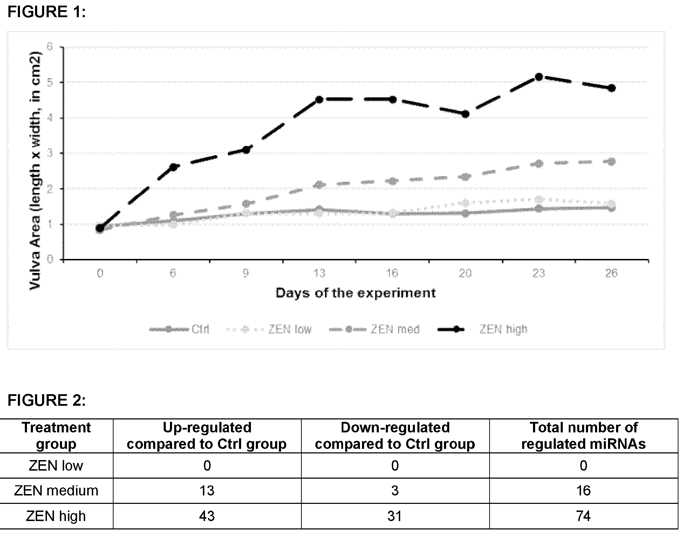

Results: Exposure to ZEN increased in a dose- and time-dependent manner the vulva surface (see FIG. 1). Specifically, piglets of the ZEN medium and ZEN high group showed a significant increase of the vulva surface after thirteen and six days of exposure, respectively. At d 26, the increase of vulva surface compared to the control group was +90% for ZEN medium and +230% for ZEN high. Similarly, the weight of the whole reproductive tract (including vagina, uterus, uterine tubes, ovaries) was significantly increased in piglets of the ZEN high group. The reproductive tract weight was expressed relative to the individual body weight ([reproductive tract (in grams)/body weight (in kg)]*100), and the calculated average (± SD) was as follows: 51.8 ± 20.6, 55.8 ± 17.2, 121.4 ± 43.4 and 353.4 ± 110.6 in the Control, ZEN low, ZEN medium and ZEN high group, respectively. Significant differences were observed between the ZEN high and control group (p-values <0.001). Independent of the concentration, ZEN exposure had no effect on the body weight of the piglets.

### Example 2: miRNA analysis in tissues

miRNA Extraction and Library Preparation: A total of 18 animals was used for investigations on the miRNA profile in the tissues: 6 from Ctrl, 4 from ZEN low, 4 from ZEN medium, and 4 from ZEN high. About 30 mg of tissue from uterus or jejunum was disrupted via a bead-beating step, and the total RNA, including RNA from approximately 18 nucleotides upwards, was extracted and purified with the miRNeasy Mini Kit (Qiagen, Hilden, Germany), following the manufacturer's instructions. The concentration of RNA was first estimated on the Nanodrop (Termo Scientifc NanoDrop 2000, USA), and again with the Agilent 2100 Bioanalyzer (Agilent Technologies, Germany) during the measurement of the RIN for determining the RNA quality. In total 1 µg of total RNA was used for construction of small RNA sequencing libraries using the NEBNext Multiplex SmallRNA Library preparation set for Illumina (New England Biolabs). Adapter-ligated total RNA was reverse transcribed into cDNA, which was used as template for PCR amplification (15 cycles) using Illumina's SR Primer and Index Primers 1-18. Barcoded DNA libraries were purified using the QIAQuick PCR purification protocol (Qiagen, Hilden, Germany) and quantified using the DNA 1000 high sensitivity chip (Agilent Technologies, Germany). Purified samples were pooled at equimolar (50 nM) concentration. Size selection was performed using gel purification to select for insert sizes between 18 and 50 nucleotides. The final multiplexed (n=18) libraries were sequenced on a NextSeq 500 sequencing instrument according to the manufacturer instructions. Raw data was de-multiplexed and FASTQ files for each sample was generated using the bcl2fastq software (Illumina inc.). FASTQ data was checked using the FastQC online tool (http://www.bioinformatics.babraham.ac.uk/projects/fastqc/).

RNA sequencing (RNA-Seq) Bioinformatics Analysis: Total reads were adapter trimmed using Cutadapt and quality filtered (Q-Score >30) using the FastQC tool. Trimmed and quality filtered reads were aligned to reference miRNA sequences in miRBase release 21 (www.mirbase.org; Kozomora and Griffiths-Jones. 2014. Nucleid Acids Research, 42, D68-D73) using bowtie2. Subsequently, reads were mapped against the *Sus scrofa* genome assembly Sscrofa10.2 as well as Ensembl small non-coding RNA reference databases. Reads were normalized to the total read count per library to generate tags per million (TPM) values. Clustvis was used for unsupervised clustering analysis. EdgeR (Robinson et al. 2010. Bioinformatics, 26, 139-140) was used for differential expression analysis based on TPM values. The p-values obtained were adjusted for multiple testing using Benjamini-Hochberg false discovery rate (FDR). Adjusted p-values ≤0.07 were considered significant.

RNA-Seq validation: Real-time polymerase chain reaction, also referred to as quantitative polymerase chain reaction (qPCR), was employed for verification of some results generated in the RNA-Seq for the uterus. Starting from the same total RNA samples used for sequencing analysis, cDNA was synthesized using the Universal cDNA Synthesis Kit II (Exiqon, Denmark) in a reverse transcription (RT) reaction using 50 ng of total RNA. Reaction conditions were used according to the manufacturer's recommendations. To monitor RT efficiency and presence of impurities with inhibitory activity, a synthetic RNA spike-in (cel-miR-39-3p) was added to the RT reaction. PCR amplification was performed in a 96-well plate format in a Roche LC480 II instrument (Roche, Germany) and using the EXiLENT SYBR Green mastermix (Exiqon, Denmark) with the following settings: 95 °C for 10 min, 45 cycles of 95 °C for 10 s and 60 °C for 60 s, followed by melting curve analysis. To calculate the cycle of quantification values (Cq-values), the second derivative method was used. Cq-values were normalized to the mean Cq-value in each sample (global mean normalization) by subtracting the individual miRNA Cq-value from the Cq average calculated for that sample.

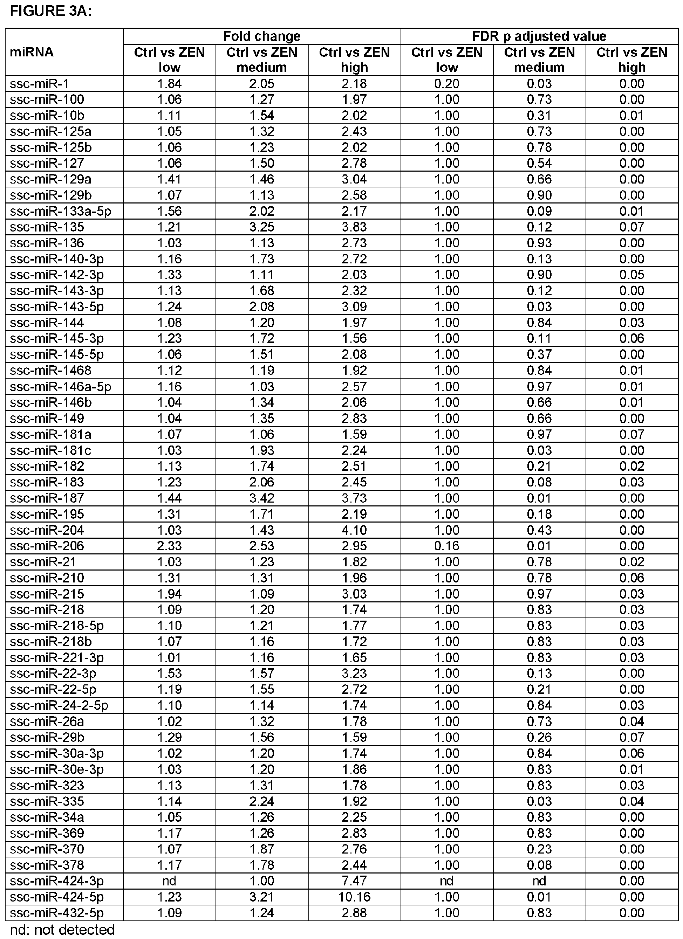

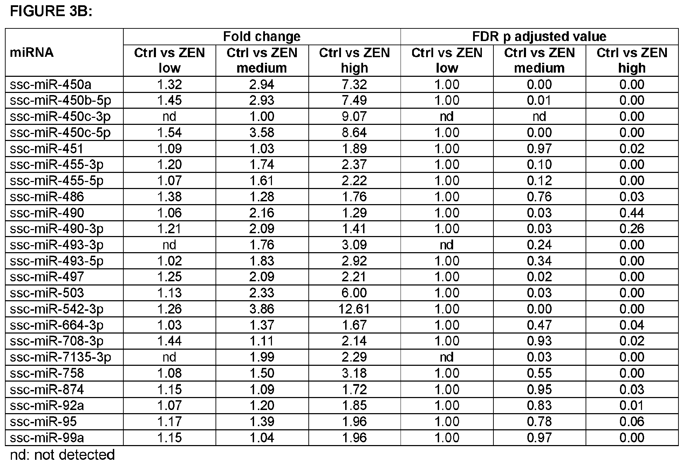

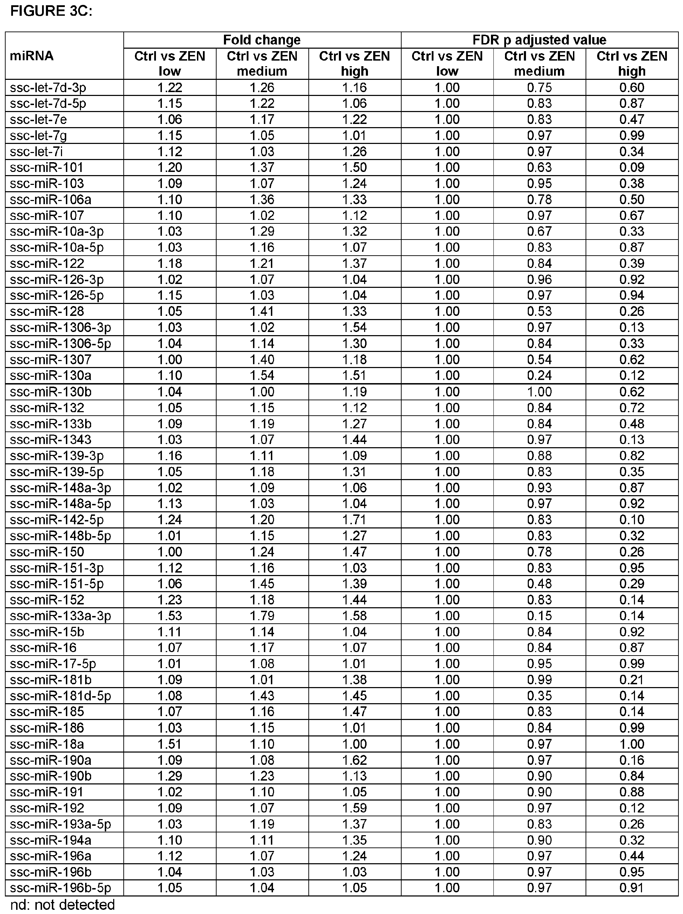

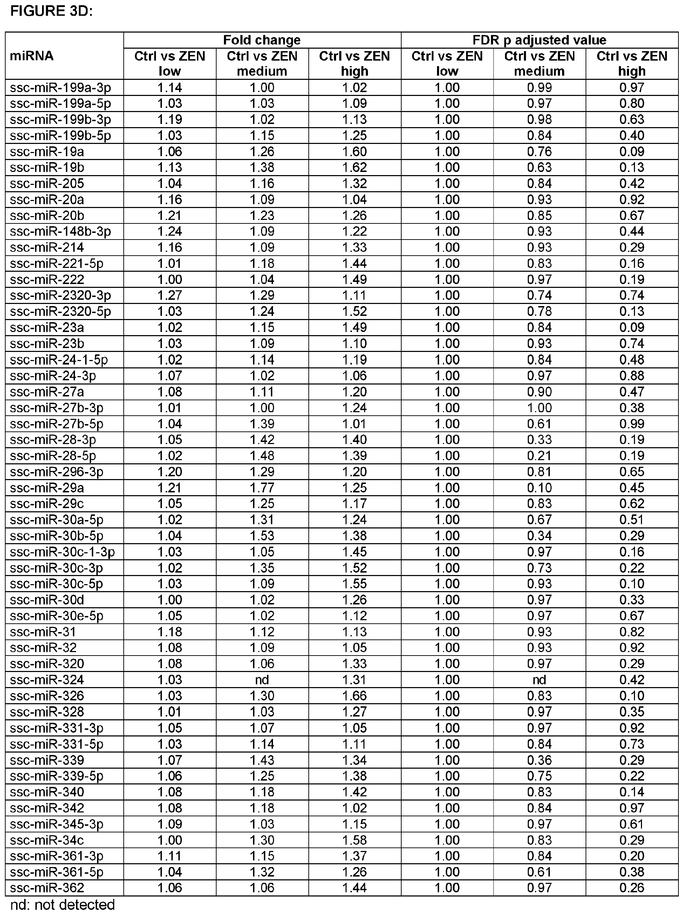

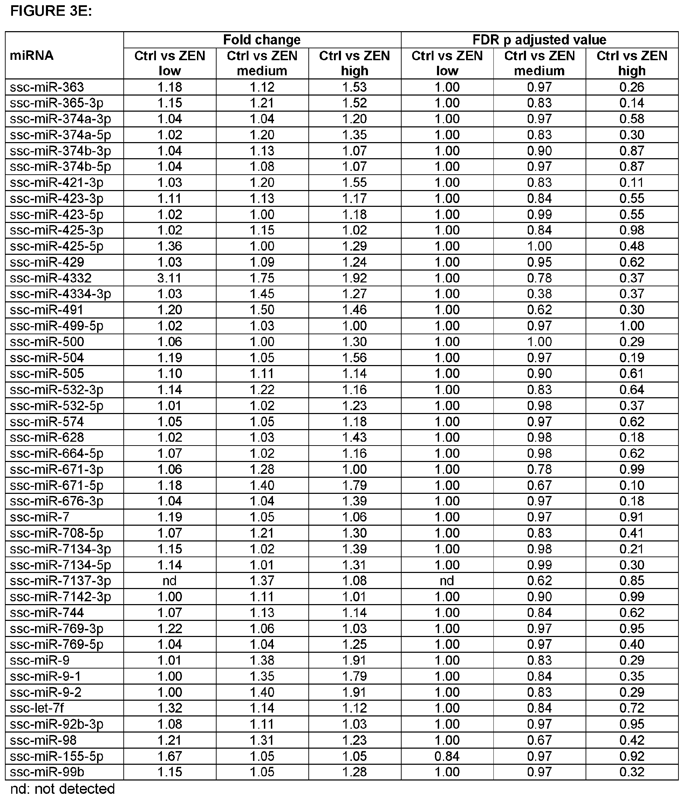

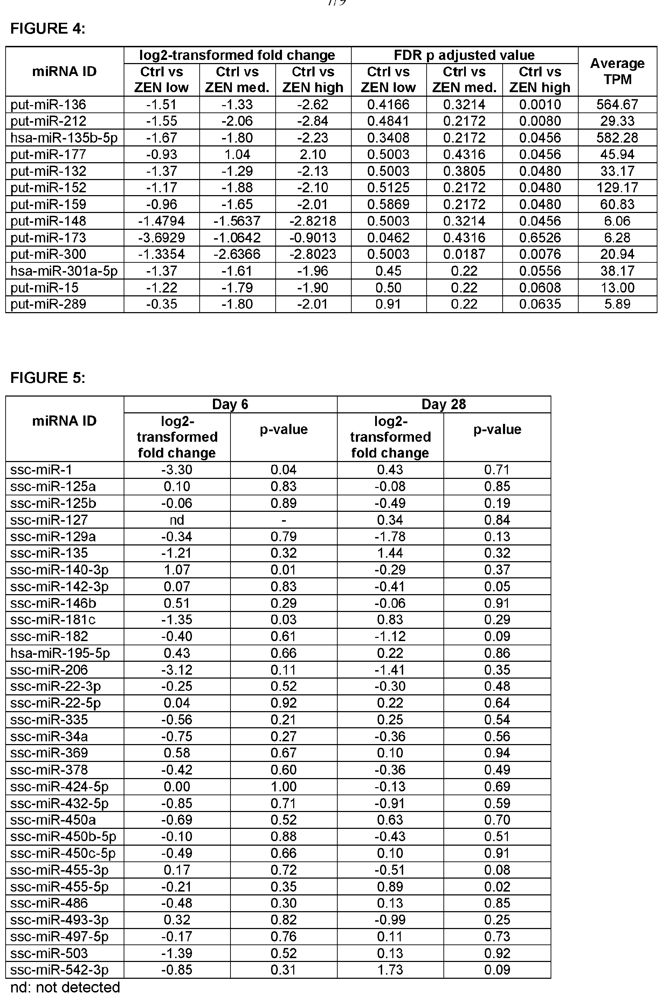

Results: Small RNA sequencing revealed the presence of 224 miRNAs (with tags per million > 1) known in pig species (ssc) in the uterus samples out of a total of 461 mature miRNAs that are currently known for pigs from miRBase. Out of those, 16 (ZEN medium) and 74 (ZEN high) miRNAs were significantly affected after ZEN exposure. FIG. 2 gives an overview on the number of up- and down-regulated miRNAs in the different treatment groups. In the ZEN medium group, fold changes of significantly affected species-specific miRNAs reached from -3.4 (ssc-miRNA-187) to +3.9 (ssc-miRNA-542-3p) compared to the control group (FIG. 3A and 3B). In the ZEN high group, effects were more pronounced with fold changes reaching from -4.1 (ssc-miRNA-204) to +12.6 (ssc-miRNA-542-3p). In total, 14 miRNAs (ssc-miR-1, ssc-miR-143-5p, ssc-miR-181c, ssc-miR-187, ssc-miR-206, ssc-miR-335, ssc-miR-424-5p, ssc-miR-450a, ssc-miR-450b-5p, ssc-miR-450c-5p, ssc-miR-497, ssc-miR-503, ssc-miR-542-3p, ssc-miR-7135-3p) were commonly affected in ZEN medium and ZEN high group. ZEN exposure led to a significant expression deviation of 13 putative or predicted miRNAs (FIG. 4). Out of those, put-miR-300 was significantly affected in ZEN medium and ZEN high. To validate the next-generation RNA sequencing results ssc-miR-542-3p, ssc-miR-450c-5p, ssc-miR-503, ssc-miR-424-5p, ssc-miR-181c were successfully quantified in uterus samples by qPCR for all groups.

### Example 3: miRNA analysis in blood serum samples

miRNA extraction and qPCR analysis: Unlike the small-RNA-Seq analysis, all the animals from the feeding trial were used for investigations of the miRNA profile in the serum (6 per group; total of 24 animals). RNA was isolated from the serum samples using the miRNeasy Mini Kit (Qiagen, Germany). Serum samples were thawed on ice and centrifuged at 12,000 x g for 5 minutes to remove any cellular debris. For each sample, 200 µL of serum was mixed with 1000 µL of Qiazol and 1 µL of synthetic spike-ins (Exiqon, Denmark). After a 10 minute incubation at room temperature, 200 µL chloroform was added to the lysates followed by cooled centrifugation at 12,000 x g for 15 minutes at 4 °C. A volume of 650 µL of the upper aqueous phase was mixed with 7 µL glycogen (50 mg/mL) to enhance precipitation. Samples were transferred to a miRNeasy mini column, and RNA was precipitated with 750 µL ethanol followed by washing with RPE and RWT buffer. RNA was eluted in 30 µL nuclease free water and stored at -80 °C until further analysis. Starting from total RNA samples, cDNA was synthesized using the Universal cDNA Synthesis Kit II (Exiqon, Denmark). Reaction conditions were chosen according to the recommendations of the manufacturer. In total 2 µL of purified total RNA was used per 10 µL reverse transcription (RT) reaction. To monitor RT efficiency and presence of impurities with inhibitory activity, a synthetic RNA spike-in (cel-miR-39-3p) was added to the RT reaction. PCR amplification was performed in a 384-well plate format using custom Pick&Mix plates (Exiqon, Denmark) in a Roche LC480 II instrument (Roche, Germany) and EXiLENT SYBR Green mastermix (Exiqon, Denmark) with the following settings: 95 °C for 10 min, 45 cycles of 95 °C for 10 s and 60 °C for 60 s, followed by melting curve analysis. To calculate the cycle of quantification values (Cq-values), the second derivative method was used, as known by a person skilled in the art. Cq-values were normalized to the mean Cq-value in each sample (global mean normalization) by subtracting the individual miRNA Cq-value from the Cq average calculated for that sample. The miRNAs selected for targeted analysis, their responses to ZEN treatment in the uterus samples and their relevances are shown in Table 1.

**Table 1: miRNAs selected for targeted analysis.**

| **miRNA ID** | **Response to ZEN treatment in uterus** | **Relevance** |
|---|---|---|
| ssc-miR-1 | Significantly increased in both ZEN medium and ZEN high group, coefficient of correlation > 0.8, log2 fold change Ctrl vs ZEN low ≥ 0.3, TPM > 10 | miRNA with significant dose-dependent effect and certain response in low dose group |
| ssc-miR-125a | Significantly decreased in ZEN high group, coefficient of correlation < 0.8, TPM > 10 | miRNA with significant effect in high dose group and relevant expression profile |
| ssc-miR-125b | Significantly decreased in ZEN high group, coefficient of correlation < -0.8, TPM > 10 | miRNA with significant effect in high dose group and relevant expression profile |
| ssc-miR-127 | Significantly increased in ZEN high group coefficient of correlation > 0.8, TPM > 10, specificity for reproductive tract | miRNA with significant effect in high dose group, relevant expression profile and certain tissue specificity |
| ssc-miR-129a | Significantly decreased in ZEN high group, coefficient of correlation < -0.8, log2 fold change Ctrl vs ZEN high < -1.5, specificity for reproductive tract | miRNA with significant effect in high dose group, relevant expression profile and certain tissue specificity |
| ssc-miR-133a-5p | Significantly increased in ZEN high group, coefficient of correlation > 0.8, log2 fold change Ctrl vs ZEN low ≥ 0.3, TPM > 10 | miRNA with significant effect in high dose group and certain response in low dose group |
| ssc-miR-135 | Significantly decreased in ZEN high group, log2 fold change change Ctrl vs ZEN high ≤ -1.5, TPM > 10 | miRNA with significant effect in high dose group and relevant expression profile |
| ssc-miR-136 | Significantly increased in ZEN high group, coefficient of correlation > 0.8, specificity for reproductive tract | miRNA with significant effect in high dose group, relevant expression profile and certain tissue specificity |
| ssc-miR-140-3p | Significantly increased in ZEN high group, coefficient of correlation > 0.8, TPM > 10 | miRNA with significant effect in high dose group and relevant expression profile |
| ssc-miR-142-3p | Significantly increased in ZEN high group, coefficient of correlation > 0.8, TPM > 10 | miRNA with significant effect in high dose group and relevant expression profile |
| ssc-miR-143-5p | Significantly increased in both ZEN medium and ZEN high group, coefficient of correlation > 0.8, log2 fold change Ctrl vs ZEN low ≥ 0.3, log2 fold change Ctrl vs ZEN high ≥-1.5, TPM > 10 | miRNA with significant dose-dependent effect and certain response in low dose group |
| ssc-miR-146b | Significantly decreased in ZEN high group, coefficient of correlation < -0.8, TPM > 10 | miRNA with significant effect in high dose group and relevant expression profile |
| ssc-miR-181c | Significantly decreased in both ZEN medium and ZEN high group, coefficient of correlation > -0.8, TPM > 10 | miRNA with significant dose-dependent effect |
| ssc-miR-182 | Significantly increased in ZEN high group coefficient of correlation > 0.8, TPM > 10 | miRNA with significant effect in high dose group and relevant expression profile |
| ssc-miR-183 | Significantly increased in ZEN high group, coefficient of correlation > 0.8, TPM > 10 | miRNA with significant effect in high dose group and relevant expression profile |
| ssc-miR-187 | Significantly decreased in both ZEN medium and ZEN high group, coefficient of correlation > -0.8, log2 fold change Ctrl vs ZEN medium ≤ -0.3, log2 fold change Crtrl vs ZEN high ≤ -1.5 | miRNA with effect in both ZEN medium and ZEN high, certain response in low dose group |
| ssc-miR-195 | Significantly increased in ZEN high group, coefficient of correlation > 0.8, log2 fold change Ctrl vs ZEN low ≥ 0.3, TPM > 10 | miRNA with significant effect in high dose group and certain response in low dose group |
| ssc-miR-204 | Significantly decreased in ZEN high group, coefficient of correlation < -0.8, log2 fold change Ctrl vs ZEN high ≤ -1.5, TPM > 10, specificity for reproductive tract | miRNA with significant effect in high dose group, certain response in low dose group and tissue specificity |
| ssc-miR-206 | Significantly increased in both ZEN medium and ZEN high group, coefficient of correlation > 0.8, log2 fold change Ctrl vs ZEN low ≥ 0.3, log2 fold change, Ctrl vs ZEN high ≥ 1.5, TPM > 10, specificity for reproductive tract | miRNA with significant dose-dependent effect, certain response in low dose group and tissue specificity |
| ssc-miR-22-3p | Significantly increased in ZEN high group, coefficient of correlation > 0.8, log2 fold change Ctrl vs ZEN low ≥ 0.3, log2 fold Ctrl vs ZEN high ≥ 1.5; TPM > 10 | miRNA with significant effect in high dose group and certain response in low dose group |
| ssc-miR-22-5p | Significantly increased in ZEN high group, coefficient of correlation > 0.8 | miRNA with significant effect in high dose group and relevant expression profile |
| ssc-miR-335 | Significantly decreased in both ZEN medium and ZEN high group, TPM > 10 | miRNA with significant effect in both ZEN medium and ZEN high |
| ssc-miR-34a | Significantly increased in ZEN high group coefficient of correlation > 0.8 | miRNA with significant effect in high dose group and relevant expression profile |
| ssc-miR-369 | Significantly increased in ZEN high group coefficient of correlation > 0.8 | miRNA with significant effect in high dose group and relevant expression profile |
| ssc-miR-378 | Significantly increased in ZEN high group coefficient of correlation > 0.8, TPM > 10 | miRNA with significant effect in high dose group and relevant expression profile |
| ssc-miR-424-5p | Significantly increased in both ZEN medium and ZEN high group, coefficient of correlation > 0.8, log2 fold change Ctrl vs ZEN high ≥ 1.5, TPM > 10, specificity for reproductive tract | miRNA with significant dose-dependent effect, certain response in low dose group and certain tissue specificity |
| ssc-miR-432-5p | Significantly increased in ZEN high group, coefficient of correlation > 0.8, log2 fold change Ctrl vs ZEN high ≥ 1.5, specificity for reproductive tract | miRNA with significant effect in high dose group, relevant expression profile and certain tissue specificity |
| ssc-miR-450a | Significantly increased in both ZEN medium and ZEN high group, coefficient of correlation > 0.8, log2 fold change Ctrl vs ZEN high ≥ 1.5, TPM > 10, specificity for reproductive tract | miRNA with significant dose-dependent effect, certain response in low dose group and certain tissue specificity |
| ssc-miR-450b-5p | Significantly increased in both ZEN medium and ZEN high group, coefficient of correlation > 0.8, log2 fold change Ctrl vs ZEN high ≥ 1.5, TPM > 10, specificity for reproductive tract | miRNA with significant dose-dependent effect, certain response in low dose group and certain tissue specificity |
| ssc-miR-450c-5p | Significantly increased in both ZEN medium and ZEN high group, coefficient of correlation > 0.8, log2 fold change Ctrl vs ZEN high ≥ 1.5, TPM > 10, specificity for reproductive tract | miRNA with significant dose-dependent effect, certain response in low dose group and certain tissue specificity |
| ssc-miR-455-3p | Significantly increased in ZEN high group, coefficient of correlation > 0.8, TPM > 10 | miRNA with significant effect in high dose group and relevant expression profile |
| ssc-miR-455-5p | Significantly increased in ZEN high group, coefficient of correlation > 0.8, TPM > 10 | miRNA with significant effect in high dose group and relevant expression profile |
| ssc-miR-486 | Significantly increased in ZEN high group, coefficient of correlation > 0.8, log2 fold change Ctrl vs ZEN low ≥ 0.3, TPM > 10 | miRNA with significant effect in high dose group and certain response in low dose group |
| ssc-miR-493-3p | Significantly increased in ZEN high group, log2 fold change Ctrl vs ZEN high ≥ 1.5, specificity for reproductive tract | miRNA with significant effect in high dose group and relevant expression pattern |
| ssc-miR-493-5p | Significantly increased in ZEN high group, coefficient of correlation > 0.8, log2 fold change Ctrl vs ZEN low ≥ 0.3, TPM > 10, specificity for reproductive tract | miRNA with significant effect in high dose group, relevant expression profile and certain tissue specificity |
| ssc-miR-497 | Significantly increased in both ZEN medium and ZEN high group, log2 fold change Ctrl vs ZEN low ≥ 0.3, TPM > 10 | miRNA with significant effect in both ZEN medium and ZEN high, certain response in ZEN low |
| ssc-miR-503 | Significantly increased in both ZEN medium and ZEN high group, coefficient of correlation > 0.8, log2 fold change Ctrl vs ZEN high ≥ 1.5, TPM > 10, specificity for reproductive tract | miRNA with significant dose-dependent effect and certain tissue specificity |
| ssc-miR-542-3p | Significantly increased in both ZEN medium and ZEN high group, coefficient of correlation > 0.8, log2 fold change Ctrl vs ZEN low ≥ 0.3, log2 fold change Ctrl vs ZEN high ≥ 1.5, TPM > 10 | miRNA with significant dose-dependent effect and certain response in low dose group |
| ssc-miR-708-3p | Significantly decreased in ZEN high group, log2 fold change Ctrl vs ZEN low ≤ -0.3, TPM > 10 | miRNA with significant effect in high dose group and certain response in low dose group |
| ssc-miR-758 | Significantly increased in ZEN high group, coefficient of correlation > 0.8, log2 fold change Ctrl vs ZEN high ≥ 1.5 | miRNA with significant effect in high dose group and relevant expression profile |
| ssc-miR-7135-3p | Significantly increased in both ZEN medium and ZEN high group | miRNA with significant effect in both ZEN medium and ZEN high group |
| hsa-miR-135b-5p | Significantly decreased in ZEN high group, coefficient of correlation < -0.8; log2 fold change Ctrl vs ZEN low ≤ -0.3, log2 fold change Ctrl vs ZEN high ≤ -1.5, TPM > 10 | miRNA with significant effect in high dose group and certain response in low dose group |
| hsa-miR-301a-5p | Significantly decreased in ZEN high group, coefficient of correlation < -0.8, log2 fold change Ctrl vs ZEN low ≤ -0.3, log2 fold change Ctrl vs ZEN high ≤ -1.5, TPM > 10 | miRNA with significant effect in high dose group, relevant expression pattern and certain response in low dose group |
| put-miR-300 | Significantly decreased in ZEN medium and high group, log2 fold change Ctrl vs ZEN low ≤ -0.3, log2 fold change Ctrl vs ZEN high ≤ -1.5, TPM > 10 | miRNA in both ZEN medium and ZEN high group, relevant expression pattern and certain response in low dose group |

Results: Targeted analysis of ssc-miR-1, ssc-miR-125a, ssc-miR-125b, ssc-miR-127, ssc-miR-129a, ssc-miR-133a-5p, ssc-miR-135, ssc-miR-136, ssc-miR-140-3p, ssc-miR-142-3p, ssc-miR-143-5p, ssc-miR-146b, ssc-miR-181c, ssc-miR-182, ssc-miR-183, ssc-miR-187, ssc-miR-195, ssc-miR-204, ssc-miR-206, ssc-miR-22-3p, ssc-miR-22-5p, ssc-miR-335, ssc-miR-34a, ssc-miR-369, ssc-miR-378, ssc-miR-424-5p, ssc-miR-432-5p, ssc-miR-450a, ssc-miR-450b-5p, ssc-miR-450c-5p, ssc-miR-455-3p, ssc-miR-455-5p, ssc-miR-486, ssc-miR-493-3p, ssc-miR-493-5p, ssc-miR-497, ssc-miR-503, ssc-miR-542-3p, ssc-miR-708-3p, ssc-miR-758, ssc-miR-7135-3p, hsa-miR-135b-5p, hsa-miR-301a-5p, put-miR-300 was performed on serum samples collected after 6 and 28 days of ZEN exposure. For ssc-miR-135, ssc-miR-187, ssc-miR-195, ssc-miR-497, amplification via qPCR was done with primers designed for the human ortholog sequences. However, sequence identity between the pig and human sequence for these four miRNA was 100%. After removing several miRNAs from the dataset due to a too low read count (ssc-miR-143-5p, ssc-miR-187, ssc-miR-493-5p, ssc-miR-708-3p, ssc-miR-7135-3p, ssc-miR-135b-5p, hsa-miR-301a-5p, put-miR-300), focus was paid on the comparison of quantifiable miRNA levels between the Ctrl group and the ZEN high group. Three (day 6) and five (day 28) miRNAs showed up- or down-regulation after ZEN exposure (unadjusted p-value ≤0.1). Up-regulated miRNAs comprise ssc-miR-140-3p, ssc-miR-455-3p, ssc-miR-455-5p and ssc-miR-542-3p, while down-regulated miRNAs include ssc-miR-1, ssc-miR-142-3p, ssc-miR-181c and ssc-miR-182. FIG. 5 gives details on the log2-transformed fold changes of serum miRNA levels after ZEN exposure. In addition to the aforementioned miRNAs, ZEN had a considerable effect on log2-transformed fold changes (≤-0.8 or ≥0.8) of six miRNAs, namely ssc-miR-129a, ssc-miR-135, ssc-miR-206, ssc-miR-432-5p, ssc-miR-493-3p and ssc-miR-503. To further explore the global effects of ZEN regardless of the concentrations, principal component analyses (PCAs) were done on the data (fold changes) for all the groups and for all the miRNAs. This allowed detection of miRNAs which are not constant across all the treatments. Hereby some miRNAs were found that were already reported to be affected, but also new ones such as ssc-miR-183. Concluding from the data generated, ssc-miR-1, ssc-miR-129a, hsa-miR-135a-3p, ssc-140-3p, ssc-miR-142-3p, ssc-miR-181c, ssc-miR-182, ssc-miR-183, ssc-206, ssc-miR-432-5p, ssc-miR-455-3p, ssc-miR-455-5p, ssc-miR-493-3p, ssc-miR-503 and ssc-miR-542-3p were identified as suitable miRNAs to be used as mechanism-based biomarkers for ZEN effect, based on significant up- or down-regulation and relevant expression pattern which considers both relative fold change and absolute expression levels.

### Example 4: Correlation of miRNA levels in tissue and serum

To compare miRNA results obtained for uterus (NGS read count) and serum (qPCR dCq values), correlation analysis was performed. To this end, NGS read counts were log2-transformed and residuals (difference to mean) were calculated and used for correlation. Two miRNAs showed a trend for correlating tissue and serum expression levels: ssc-miR-455-5p (sample size: 17; correlation coefficient r=0.4045; significance level P=0.1073; 95% confidence interval for r: -0.09449 to 0.7411) and ssc-miR-542-3p (sample size: 17; correlation coefficient r=0.4244; significance level P=0.0895; 95% confidence interval for r: -0.07066 tot 0.7517).

### Example 5: miRNA ratios and Receiver Operating Characteristic (ROC) analysis

Ratios between selected miRNAs identified to be affected by ZEN exposure in the serum samples were established and subsequently subjected to the ROC (Receiver Operating Characteristic) approach as a validated and common method for evaluation of biomarkers (Grund and Sabin. 2010. Current Opinion in HIV and AIDS, 5, 473-479). Hereby, suitable ratios were identified that allowed discrimination between pigs non-exposed to ZEN (Ctrl) and pigs exposed to ZEN (ZEN low, ZEN medium, ZEN high): ssc-miR-135/ssc-miR-432-5p, ssc-miR-455-5p/ssc-miR-493-3p, ssc-miR-542-3p/ssc-miR-1 and ssc-miR-542-3p/ssc-miR-493-3p (FIG. 6).

In addition to the 15 serum miRNAs identified as suitable miRNAs to be used as mechanism-based biomarkers for ZEN effect described in Example 3, the miRNA ratios ssc-miR-135/ssc-miR-432-5p, ssc-miR-455-5p/ssc-miR-493-3p, ssc-miR-542-3p/ssc-miR-1 and ssc-miR-542-3p/ssc-miR-493-3p were found to be especially reliable and well-pronounced biomarkers to allow a detection of ZEN effect.

## Claims

1. A method for detecting zearalenone effect, wherein zearalenone effect refers to exposure of a biological system to zearalenone or an effect of zearalenone on a biological system, comprising:
(c) determining in a test sample the expression level of at least one miRNA selected from the group consisting of ssc-miR-1, ssc-miR-181c, ssc-miR-206, ssc-miR-503, ssc-miR-542-3p, ssc-miR-135, ssc-miR-129a-3p, ssc-miR-142-3p, ssc-miR-432-5p, ssc-miR-455-5p, ssc-miR-182, ssc-miR-493-3p, ssc-miR-455-3p, ssc-miR-183, and ssc-miR-140-3p, wherein the test sample is a blood sample; and
(d) comparing the expression level with a reference value.

2. The method of claim 1, wherein the reference value corresponds to the expression level of said at least one miRNA in a control sample.

3. The method of claim 1, wherein the expression level of at least one miRNA comprises at least one ratio of expression levels between two miRNAs selected from the group consisting of ssc-miR-542-3p and ssc-miR-1, ssc-miR-542-3p and ssc-miR-493-3p, ssc-miR-135 and ssc-miR-432-5p, and ssc-miR-455-5p and ssc-miR-493-3p.

4. The method of any one of the preceding claims, wherein the test sample has been obtained from a subject, wherein the subject is preferably a mammal, preferably of the genus *Sus,* preferably of the species *Sus scrofa.*

5. The method of any one of the preceding claims further comprising
(b) providing the test sample; and/or
(e) providing information regarding zearalenone effect.

6. The method of any one of the preceding claims, wherein the method is used in a method for diagnosing zearalalenone exposure.

7. The method of any one of claims 1-5, wherein the method is used in a method for detecting the presence of zearalenone in feed or food, wherein the method is preferably for selecting feed for contacting with a zearalenone neutralizing agent or applying a zearalenone neutralizing method.

8. The method of any one of claims 4-7, comprising
(a) feeding the subject with feed suspected to contain zearalenone prior to obtaining the test sample from the subject; and/or
(f) selecting the feed for contacting with a zearalenone neutralizing agent or applying a zearalenone neutralizing method if a deviation in the expression levels of the at least one miRNA between the test sample and the control sample indicates zearalenone exposure.

9. The method of any one of the preceding claims, wherein the method is used in a method for assessing the capacity of a method or test compound to neutralize zearalenone.

10. Use of at least one miRNA selected from the group consisting of miR-1, miR-181c, miR-206, miR-503, miR-542-3p, miR-135, miR-135a-5p, miR-129a-3p, miR-142-3p, miR-432-5p, miR-455-5p, miR-182, miR-493-3p, miR-455-3p, miR-183, and miR-140-3p, for detecting zearalenone effect in a test sample, wherein zearalenone effect refers to exposure of a biological system to zearalenone or an effect of zearalenone on a biological system, wherein the test sample is a blood sample.

11. A data processing system comprising a processor configured to perform a method comprising the steps of
(a) comparing the expression level of at least one miRNA selected from the group consisting of miR-1, miR-181c, miR-206, miR-503, miR-542-3p, miR-135, miR-135a-5p, miR-129a-3p, miR-142-3p, miR-432-5p, miR-455-5p, miR-182, miR-493-3p, miR-455-3p, miR-183, and miR-140-3p, determined in a test sample obtained from a subject with a reference value, wherein the test sample is a blood sample; and
(b) indicating zearalenone exposure of the subject if a deviation is determined, wherein the reference value corresponds to the expression level that has been determined in a control sample that has been obtained from a subject that has not been exposed to zearalenone; or indicating zearalenone exposure of the subject if no deviation is determined, wherein the reference value corresponds to the expression level that has been determined in a control sample that has been obtained from a subject that has been exposed to zearalenone.

12. A sequencing device capable of determining the level of at least one miRNA selected from the group consisting of miR-1, miR-181c, miR-206, miR-503, miR-542-3p, miR-135, miR-135a-5p, miR-129a-3p, miR-142-3p, miR-432-5p, miR-455-5p, miR-182, miR-493-3p, miR-455-3p, miR-183, and miR-140-3p, comprising the data processing system of claim 11.

13. A computer program comprising instructions to cause the data processing system of claim 11 or the sequencing device of claim 12 to execute the steps of
(b) comparing the expression level of at least one miRNA selected from the group consisting of miR-1, miR-181c, miR-206, miR-503, miR-542-3p, miR-135, miR-135a-5p, miR-129a-3p, miR-142-3p, miR-432-5p, miR-455-5p, miR-182, miR-493-3p, miR-455-3p, miR-183, and miR-140-3p, determined in a sample obtained from a subject, wherein the sample is a blood sample, with a reference value; and
(c) indicating zearalenone exposure of the subject if a deviation is determined, wherein the reference value corresponds to the expression level that has been determined in a control sample that has been obtained from a subject that has not been exposed to zearalenone; or indicating zearalenone exposure of the subject if no deviation is determined, wherein the reference value corresponds to the expression level that has been determined in a control sample that has been obtained from a subject that has been exposed to zearalenone; or
a computer-readable medium having stored thereon the computer program.

14. Use of a kit for performing the method of any one of claims 1-9, wherein the kit comprisies a specific binding agent for at least one miRNA selected from the group consisting of miR-1, miR-181c, miR-206, miR-503, miR-542-3p, miR-135, miR-135a-5p, miR-129a-3p, miR-142-3p, miR-432-5p, miR-455-5p, miR-182, miR-493-3p, miR-455-3p, miR-183, and miR-140-3p, wherein the specific binding agent is conjugated to a solid support.

## Patentansprüche

1. Verfahren zum Nachweis eines Zearalenon-Effekts, wobei sich der Zearalenon-Effekt auf die Exposition eines biologischen Systems gegenüber Zearalenon oder einen Effekt von Zearalenon auf ein biologisches System bezieht, umfassend:
(c) Bestimmen des Expressionsniveaus mindestens einer miRNA in einer Testprobe, ausgewählt aus der Gruppe bestehend aus ssc-miR-1, ssc-miR-181c, ssc-miR-206, ssc-miR-503, ssc-miR-542-3p, ssc-miR-135, ssc-miR-129a-3p, ssc-miR-142-3p, ssc-miR-432-5p, ssc-miR-455-5p, ssc-miR-182, ssc-miR-493-3p, ssc-miR-455-3p, ssc-miR-183, und ssc-miR-140-3p, wobei die Testprobe eine Blutprobe ist; und
(d) Vergleichen des Expressionsniveaus mit einem Referenzwert.

2. Verfahren nach Anspruch 1, wobei der Referenzwert dem Expressionsniveau der mindestens einen miRNA in einer Kontrollprobe entspricht.

3. Verfahren nach Anspruch 1, wobei das Expressionsniveau mindestens einer miRNA mindestens ein Verhältnis der Expressionsniveaus zwischen zwei miRNAs, ausgewählt aus der Gruppe bestehend aus ssc-miR-542-3p und ssc-miR-1, ssc-miR-542-3p und ssc-miR-493-3p, ssc-miR-135 und ssc-miR-432-5p und ssc-miR-455-5p und ssc-miR-493-3p, umfasst.

4. Verfahren nach einem der vorhergehenden Ansprüche, wobei die Testprobe von einem Subjekt erhalten wurde, wobei das Subjekt vorzugsweise ein Säugetier ist, vorzugsweise der Gattung *Sus,* vorzugsweise der Spezies *Sus scrofa.*

5. Das Verfahren nach einem der vorangehenden Ansprüche ferner umfassend
(b) Bereitstellen der Testprobe; und/oder
(e) Bereitstellung von Informationen über den Zearalenon-Effekt.

6. Verfahren nach einem der vorangehenden Ansprüche, wobei das Verfahren in einem Verfahren zur Diagnose einer Zearalenon-Exposition verwendet wird.

7. Verfahren nach einem der Ansprüche 1 bis 5, wobei das Verfahren in einem Verfahren zum Nachweis des Vorhandenseins von Zearalenon in Futter- oder Nahrungsmitteln verwendet wird, wobei das Verfahren vorzugsweise der Auswahl von Futtermitteln für den Kontakt mit einem Zearalenon-Neutralisierungsmittel oder der Anwendung eines Zearalenon-Neutralisierungsverfahrens dient.

8. Verfahren nach einem der Ansprüche 4-7, umfassend
(a) Füttern des Subjekts mit einem Futtermittel, von dem vermutet wird, dass es Zearalenon enthält, bevor die Testprobe von dem Subjekt entnommen wird; und/oder
(f) Auswahl des Futters für das Inkontaktbringen mit einem Zearalenon-Neutralisierungsmittel oder die Anwendung eines Zearalenon-Neutralisierungsverfahrens, wenn eine Abweichung in den Expressionsniveaus der mindestens einen miRNA zwischen der Testprobe und der Kontrollprobe auf eine Zearalenon-Exposition hinweist.

9. Verfahren nach einem der vorhergehenden Ansprüche, wobei das Verfahren in einem Verfahren zur Bewertung der Fähigkeit eines Verfahrens oder einer Testverbindung, Zearalenon zu neutralisieren, verwendet wird.

10. Verwendung von mindestens einer miRNA, ausgewählt aus der Gruppe bestehend aus miR-1, miR-181c, miR-206, miR-503, miR-542-3p, miR-135, miR-135a-5p, miR-129a-3p, miR-142-3p, miR-432-5p, miR-455-5p, miR-182, miR-493-3p, miR-455-3p, miR-183 und miR-140-3p, zum Nachweis eines Zearalenon-Effekts in einer Testprobe, wobei sich der Zearalenon-Effekt auf die Exposition eines biologischen Systems gegenüber Zearalenon oder einen Effekt von Zearalenon auf ein biologisches System bezieht, wobei die Testprobe eine Blutprobe ist.

11. Datenverarbeitungssystem, das einen Prozessor umfasst, der so konfiguriert ist, dass er ein Verfahren durchführt, das die folgenden Schritte umfasst
(a) Vergleichen des Expressionsniveaus von mindestens einer miRNA, ausgewählt aus der Gruppe bestehend aus miR-1, miR-181c, miR-206, miR-503, miR-542-3p, miR-135, miR-135a-5p, miR-129a-3p, miR-142-3p, miR-432-5p, miR-455-5p, miR-182, miR-493-3p, miR-455-3p, miR-183 und miR-140-3p, das in einer von einem Probanden erhaltenen Testprobe bestimmt wurde, mit einem Referenzwert, wobei die Testprobe eine Blutprobe ist; und
(b) Anzeigen einer Zearalenon-Exposition des Subjekts, wenn eine Abweichung bestimmt wird, wobei der Referenzwert dem Expressionsniveau entspricht, das in einer Kontrollprobe bestimmt wurde, die von einem Subjekt gewonnen wurde, das nicht Zearalenon ausgesetzt war; oder Anzeigen der Zearalenon-Exposition des Subjekts, wenn keine Abweichung bestimmt wird, wobei der Referenzwert dem Expressionsniveau entspricht, das in einer Kontrollprobe bestimmt wurde, die von einem Subjekt gewonnen wurde, das Zearalenon ausgesetzt war.

12. Sequenziervorrichtung, die in der Lage ist, das Niveau von mindestens einer miRNA zu bestimmen, die ausgewählt ist aus der Gruppe bestehend aus miR-1, miR-181c, miR-206, miR-503, miR-542-3p, miR-135, miR-135, miR-135a-5p, miR-129a-3p, miR-142-3p, miR-432-5p, miR-455-5p, miR-182, miR-493-3p, miR-455-3p, miR-183 und miR-140-3p, umfassend das Datenverarbeitungssystem nach Anspruch 11.

13. Computerprogramm, das Anweisungen umfasst, um das Datenverarbeitungssystem nach Anspruch 11 oder die Sequenziervorrichtung nach Anspruch 12 zu veranlassen, die folgenden Schritte auszuführen
(b) Vergleichen des Expressionsniveaus von mindestens einer miRNA, ausgewählt aus der Gruppe bestehend aus miR-1, miR-181c, miR-206, miR-503, miR-542-3p, miR-135, miR-135a-5p, miR-129a-3p, miR-142-3p, miR-432-5p, miR-455-5p, miR-182, miR-493-3p, miR-455-3p, miR-183 und miR-140-3p, das in einer von einem Probanden erhaltenen Testprobe bestimmt wurde, wobei die Probe eine Blutprobe ist, mit einem Referenzwert; und
(c) Anzeigen der Zearalenon-Exposition des Subjekts, wenn eine Abweichung bestimmt wird, wobei der Referenzwert dem Expressionsniveau entspricht, das in einer Kontrollprobe bestimmt wurde, die von einem Subjekt gewonnen wurde, das nicht Zearalenon ausgesetzt war; oder Anzeigen der Zearalenon-Exposition des Subjekts, wenn keine Abweichung bestimmt wird, wobei der Referenzwert dem Expressionsniveau entspricht, das in einer Kontrollprobe bestimmt wurde, die von einem Subjekt gewonnen wurde, das Zearalenon ausgesetzt war; oder
ein computerlesbares Medium, auf dem das Computerprogramm gespeichert ist.

14. Verwendung eines Kits zur Durchführung des Verfahrens nach einem der Ansprüche 1-9, wobei das Kit ein spezifisches Bindemittel für mindestens eine miRNA, ausgewählt aus der Gruppe bestehend aus miR-1, miR-181c, miR-206, miR-503, miR-542-3p, miR-135, miR-135a-5p, miR-129a-3p, miR-142-3p, miR-432-5p, miR-455-5p, miR-182, miR-493-3p, miR-455-3p, miR-183 und miR-140-3p umfasst, wobei das spezifische Bindungsmittel mit einem festen Träger konjugiert ist.

## Revendications

1. Méthode de détection de l'effet de la zéaralénone, dans laquelle l'effet de la zéaralénone fait référence à l'exposition d'un système biologique à la zéaralénone ou à un effet de la zéaralénone sur un système biologique, comprenant:
(c) déterminer dans un échantillon de test le niveau d'expression d'au moins un miARN choisi dans le groupe constitué de ssc-miR-1, ssc-miR-181c, ssc-miR-206, ssc-miR-503, ssc-miR-542-3p, ssc-miR-135, ssc-miR-129a-3p, ssc-miR-142-3p, ssc-miR-432-5p, ssc-miR-455-5p, ssc-miR-182, ssc-miR-493-3p, ssc-miR-455-3p, ssc-miR-183 et ssc-miR-140-3p, l'échantillon de test étant un échantillon de sang; et
(d) comparer le niveau d'expression à une valeur de référence.

2. Méthode de la revendication 1, dans laquelle la valeur de référence correspond au niveau d'expression dudit au moins un miARN dans un échantillon de contrôle.

3. Méthode de la revendication 1, dans laquelle le niveau d'expression d'au moins un miARN comprend au moins un rapport des niveaux d'expression entre deux miARN choisis dans le groupe constitué de ssc-miR-542-3p et ssc-miR-1, ssc-miR-542-3p et ssc-miR-493-3p, ssc-miR-135 et ssc-miR-432-5p, et ssc-miR-455-5p et ssc-miR-493-3p.

4. Méthode de l'une quelconque des revendications précédentes, dans laquelle l'échantillon de test a été obtenu à partir d'un sujet, le sujet étant de préférence un mammifère, de préférence du genre *Sus,* de préférence de l'espèce *Sus scrofa.*

5. Méthode de l'une quelconque des revendications précédentes, comprenant en outre
(b) fournir l'échantillon de test; et/ou
(e) fournir des informations concernant l'effet de la zéaralénone.

6. Méthode de l'une quelconque des revendications précédentes, dans laquelle la méthode est utilisée dans une méthode de diagnostic de l'exposition à la zéaralénone.

7. Méthode de l'une des revendications 1 à 5, dans laquelle la méthode est utilisée dans une méthode de détection de la présence de zéaralénone dans les aliments pour animaux ou les denrées alimentaires, la méthode étant de préférence utilisée pour sélectionner les aliments pour animaux à mettre en contact avec un agent neutralisant la zéaralénone ou pour appliquer une méthode de neutralisation de la zéaralénone.

8. Méthode de l'une des revendications 4 à 7, comprenant
(a) nourrir le sujet avec des aliments soupçonnés de contenir de la zéaralénone avant d'obtenir l'échantillon de test du sujet ; et/ou
(f) sélectionner l'aliment pour le mettre en contact avec un agent neutralisant la zéaralénone ou appliquer une méthode de neutralisation de la zéaralénone si un écart dans les niveaux d'expression d'au moins un miARN entre l'échantillon d'essai et l'échantillon de contrôle indique une exposition à la zéaralénone.

9. Méthode de l'une quelconque des revendications précédentes, dans laquelle la méthode est utilisée dans une méthode d'évaluation de la capacité d'une méthode ou d'un composé d'essai à neutraliser la zéaralénone.

10. Utilisation d'au moins un miARN choisi dans le groupe constitué de miR-1, miR-181c, miR-206, miR-503, miR-542-3p, miR-135, miR-135a-5p, miR-129a-3p, miR-142-3p, miR-432-5p, miR-455-5p, miR-182, miR-493-3p, miR-455-3p, miR-183 et miR-140-3p, pour détecter l'effet de la zéaralénone dans un échantillon de test, l'effet de la zéaralénone faisant référence à l'exposition d'un système biologique à la zéaralénone ou à un effet de la zéaralénone sur un système biologique, l'échantillon de test étant un échantillon de sang.

11. Système de traitement des données comprenant un processeur configuré pour exécuter une méthode comprenant les étapes suivantes
(a) comparer le niveau d'expression d'au moins un miARN choisi dans le groupe constitué de miR-1, miR-181c, miR-206, miR-503, miR-542-3p, miR-135, miR-135a-5p, miR-129a-3p, miR-142-3p, miR-432-5p, miR-455-5p, miR-182, miR-493-3p, miR-455-3p, miR-183 et miR-140-3p, déterminés dans un échantillon de test obtenu à partir d'un sujet ayant une valeur de référence, l'échantillon de test étant un échantillon de sang ; et
(b) indiquer l'exposition du sujet à la zéaralénone si un écart est déterminé, la valeur de référence correspondant au niveau d'expression déterminé dans un échantillon de contrôle provenant d'un sujet qui n'a pas été exposé à la zéaralénone ; ou indiquer l'exposition du sujet à la zéaralénone si aucun écart n'est déterminé, la valeur de référence correspondant au niveau d'expression déterminé dans un échantillon de contrôle provenant d'un sujet qui a été exposé à la zéaralénone.

12. Dispositif de séquençage capable de déterminer le niveau d'au moins un miARN choisi dans le groupe constitué de miR-1, miR-181c, miR-206, miR-503, miR-542-3p, miR-135, miR-135, miR-135a-5p, miR-129a-3p, miR-142-3p, miR-432-5p, miR-455-5p, miR-182, miR-493-3p, miR-455-3p, miR-183 et miR-140-3p, en utilisant le système de traitement des données de la revendication 11.

13. Programme informatique comprenant des instructions pour amener le système de traitement des données de la revendication 11 ou le dispositif de séquençage de la revendication 12 à exécuter les étapes suivantes
(b) comparer le niveau d'expression d'au moins un miARN choisi dans le groupe constitué de miR-1, miR-181c, miR-206, miR-503, miR-542-3p, miR-135, miR-135a-5p, miR-129a-3p, miR-142-3p, miR-432-5p, miR-455-5p, miR-182, miR-493-3p, miR-455-3p, miR-183 et miR-140-3p, déterminés dans un échantillon obtenu à partir d'un sujet, l'échantillon étant un échantillon de sang, avec une valeur de référence ; et
(c) indiquer l'exposition du sujet à la zéaralénone si un écart est déterminé, la valeur de référence correspondant au niveau d'expression qui a été déterminé dans un échantillon de contrôle provenant d'un sujet qui n'a pas été exposé à la zéaralénone ; ou indiquer l'exposition du sujet à la zéaralénone si aucun écart n'est déterminé, la valeur de référence correspondant au niveau d'expression qui a été déterminé dans un échantillon de contrôle provenant d'un sujet qui a été exposé à la zéaralénone ; ou
un support lisible par ordinateur sur lequel est stocké le programme informatique.

14. Utilisation d'un kit pour la mise en œuvre de la méthode de l'une des revendications 1 à 9, dans lequel le kit comprend un agent de liaison spécifique pour au moins un miARN choisi dans le groupe constitué de miR-1, miR-181c, miR-206, miR-503, miR-542-3p, miR-135, miR-135a-5p, miR-129a-3p, miR-142-3p, miR-432-5p, miR-455-5p, miR-182, miR-493-3p, miR-455-3p, miR-183 et miR-140-3p, dans lequel l'agent de liaison spécifique est conjugué à un support solide.
